(19)

Europäisches
Patentamt
European
Patent Office
Office européen
des brevets

(11) **EP 4 269 590 A1**

(12) **EUROPEAN PATENT APPLICATION**
published in accordance with Art. 153(4) EPC

(43) Date of publication:
**01.11.2023 Bulletin 2023/44**

(21) Application number: **21910858.6**

(22) Date of filing: **22.12.2021**

(51) International Patent Classification (IPC):
*C12N 15/31* (2006.01)   *C07K 1/16* (2006.01)
*C07K 14/31* (2006.01)   *C07K 17/02* (2006.01)
*C12N 1/15* (2006.01)   *C12N 1/19* (2006.01)
*C12N 1/21* (2006.01)   *C12N 5/10* (2006.01)
*C12N 15/63* (2006.01)   *C12P 21/02* (2006.01)

(52) Cooperative Patent Classification (CPC):
**C07K 1/16; C07K 14/195; C07K 14/31;
C07K 17/02; C12N 5/10; C12N 15/63; C12N 15/74;
C12N 15/80; C12N 15/81; C12P 21/02**

(86) International application number:
**PCT/JP2021/047570**

(87) International publication number:
**WO 2022/138718 (30.06.2022 Gazette 2022/26)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **22.12.2020 JP 2020212695**

(71) Applicant: **Protenova Co., Ltd.
Kagawa 7692604 (JP)**

(72) Inventors:
• **MAJIMA, Eiji
Higahikagawa-shi, Kagawa 769-2604 (JP)**
• **SHIMA, Atsushi
Higahikagawa-shi, Kagawa 769-2604 (JP)**

(74) Representative: **Müller-Boré & Partner
Patentanwälte PartG mbB
Friedenheimer Brücke 21
80639 München (DE)**

Remarks:
The complete document including Reference
Table(s) and the Sequence Listing(s) can be
downloaded from the EPO website

(54) **IMMUNOGLOBULIN-BINDING POLYPEPTIDE**

(57)    An object of the present invention is to provide a polypeptide having excellent alkaline stability and also allowing an immunoglobulin or a fragment thereof that has been bound to be eluted in a weakly acidic range, by modifying an amino acid sequence of an immunoglobulin-binding domain of Protein A. By substitution of serine at position 41 with an amino acid with a hydrophobic side chain, tyrosine, or histidine in each immunoglobulin-binding domain of Protein A, a polypeptide is obtained having avidity for an immunoglobulin or a polypeptide containing an Fc region thereof, having excellent alkaline stability, and also allowing the immunoglobulin or the polypeptide containing an Fc region thereof that has been bound to be efficiently eluted under weakly acidic mild conditions.

EP 4 269 590 A1

**Description**

Technical Field

The present invention relates to a polypeptide that binds to an immunoglobulin or a polypeptide containing an Fc region thereof. More specifically, the present invention relates to a polypeptide having avidity for an immunoglobulin or a polypeptide containing an Fc region thereof, having excellent stability under alkaline conditions (alkaline stability), and also allowing an immunoglobulin or a fragment thereof that has been bound to be efficiently eluted in a weakly acidic range. Furthermore, the present invention relates to a method for producing the polypeptide, an immobilized product of the polypeptide, and a method for separating an immunoglobulin or a fragment thereof using the polypeptide.

Background Art

Immunoglobulins (also referred to as antibodies), which have high binding specificities for target substances, have heretofore been extensively used as research reagents or clinical test reagents. In recent years, advances in genetic recombination technology have led to the establishment of technologies for producing human antibodies or humanized antibodies, and immunoglobulins have been put to practical use as antibody preparations in medical fields such as treatment of rheumatism and cancer.

Immunoglobulins are typically produced by animal cell culture. For the purification of immunoglobulins, affinity chromatography, which uses ligands having an immunoglobulin-binding capacity, is extensively used. Affinity chromatography used for the purification of immunoglobulins uses polypeptides such as Protein A, Protein L, and Protein G or immunoglobulin-binding domains thereof as ligands that specifically bind to the immunoglobulins.

In recent years, ligands used for the purification of immunoglobulins have been required to have improved stability. In particular, in the purification of immunoglobulins, an alkaline solution is used for purposes such as inactivation of viruses or the like and washing of a carrier having a ligand immobilized thereon, and therefore one of important factors for a ligand used in the purification of immunoglobulins is to have high stability towards alkalis.

Active research has heretofore been conducted on technologies for improving the alkaline stability of Protein A. Patent Literature 1, for example, has reported that substitution of specific amino acids in the C domain of Protein A or specific amino acid residues in the Z domain of Protein A can impart an excellent immunoglobulin-binding capacity and excellent alkaline stability. Patent Literature 2 has reported that substitution of an asparagine residue of Protein A with another amino acid can impart alkaline stability.

To elute immunoglobulins bound to Protein A or immunoglobulin-binding domains thereof, a strongly acidic eluate (pH: about 3 or less) is usually used. The use of such a strongly acidic eluate may invite denaturation of immunoglobulins, accompanied by aggregate formation. Thus, there is a desire to develop an immunoglobulin-binding polypeptide allowing bound immunoglobulins to be eluted under weakly acidic mild conditions.

As stated above, although various technologies have been reported for imparting alkaline stability to Protein A and immunoglobulin-binding domains thereof, sufficient research has not so far been made on technologies for efficiently eluting bound immunoglobulins or fragments thereof in a weakly acidic range.

Citation List

Patent Literature

Patent Literature 1: WO 2007/097361
Patent Literature 2: WO 2000/023580

Summary of Invention

Technical Problem

An object of the present invention is to provide a polypeptide having excellent alkaline stability and also allowing an immunoglobulin or a polypeptide containing an Fc region thereof that has been bound to be eluted in a weakly acidic range, by modifying an amino acid sequence of an immunoglobulin-binding domain of Protein A.

Solution to Problem

The inventors of the present invention have conducted extensive research to solve the aforementioned problem, and found that, by substitution of serine at position 41 with an amino acid with a hydrophobic side chain, tyrosine, or histidine in each immunoglobulin-binding domain of Protein A, a polypeptide is obtained having avidity for an immunoglobulin or a polypeptide containing an Fc region thereof, having excellent alkaline stability, and also allowing the immunoglobulin or the polypeptide containing an Fc region thereof that has been bound to be efficiently eluted under weakly acidic mild conditions. Furthermore, the present inventors have found that, in addition to the substitution at position 41, by substitution of glutamine at position 9 with an amino acid with a hydrophobic aliphatic side chain or histidine, or by substitution of glutamic acid at position 15 with alanine or histidine and substitution of glutamic acid or alanine at position 24 with glutamine, the alkaline stability and the elution properties in a weakly acidic range can be further improved. The present invention has been completed as a result of further research based on the findings.

In summary, the present invention provides aspects of invention as itemized below:

Item 1. A polypeptide comprising at least one immunoglobulin-binding domain as set forth in any of (A) to (C):

(A) an immunoglobulin-binding domain comprising an amino acid sequence having a modification meeting the following conditions (i) to (iv) introduced into an amino acid sequence as set forth in any of SEQ ID NOS: 1 to 15:

(i) serine at position 41 is substituted with an amino acid with a hydrophobic side chain, tyrosine, or histidine;
(ii) glutamine at position 9 is unsubstituted or substituted with an amino acid with a hydrophobic aliphatic side chain or histidine;
(iii) glutamic acid or glutamine at position 15 is unsubstituted or substituted with alanine, histidine, tyrosine, or leucine; and
(iv) glutamic acid or alanine at position 24 is unsubstituted, or substituted with glutamine, histidine, or alanine in the case of SEQ ID NOS: 1 to 12, or substituted with glutamine or histidine in the case of SEQ ID NOS: 13 to 15;

(B) an immunoglobulin-binding domain having a modification meeting the conditions (i) to (iv) introduced into an amino acid sequence as set forth in any of SEQ ID NOS: 1 to 15, wherein one or a few amino acids at sites without the modification are substituted, added, inserted, and/or deleted, and wherein the immunoglobulin-binding domain has equivalent or higher alkaline stability and higher IgG elution capacity in a weakly acidic range, as compared to a polypeptide consisting of the corresponding unmodified amino acid sequence; and
(C) an immunoglobulin-binding domain having a modification meeting the conditions (i) to (iv) introduced into an amino acid sequence as set forth in any of SEQ ID NOS: 1 to 15, wherein sequence identity of sites without the modification with respect to the corresponding unmodified amino acid sequence is 80% or more, and wherein the immunoglobulin-binding domain has equivalent or higher alkaline stability and higher IgG elution capacity in a weakly acidic range, as compared to a polypeptide consisting of the corresponding unmodified amino acid sequence.

Item 2. The polypeptide according to item 1, which is a single domain peptide comprising one immunoglobulin-binding domain selected from the immunoglobulin-binding domains as set forth in (A) to (C).

Item 3. The polypeptide according to item 1, which is a multidomain peptide wherein two or more immunoglobulin-binding domains selected from the immunoglobulin-binding domains as set forth in (A) to (C) are linked.

Item 4. The polypeptide according to any one of items 1 to 3, wherein glutamine at position 9 is substituted with an amino acid with a hydrophobic aliphatic side chain or histidine in the amino acid sequence.

Item 5. The polypeptide according to any one of items 1 to 4, wherein glutamic acid or glutamine at position 15 is substituted with alanine or histidine in the amino acid sequence, and glutamic acid at position 24 is substituted with glutamine in the case of SEQ ID NOS: 1 to 12, or alanine at position 24 is substituted with glutamine in the case of SEQ ID NOS: 13 to 15.

Item 6. The polypeptide according to any one of items 1 to 5, wherein serine at position 41 is substituted with alanine, valine, leucine, phenylalanine, tyrosine, or histidine in the amino acid sequence.

Item 7. The polypeptide according to any one of items 1 to 6, wherein glutamine at position 9 is substituted with alanine, valine, leucine, isoleucine, or histidine in the amino acid sequence.

Item 8. DNA encoding the polypeptide according to any one of items 1 to 7.

Item 9. A recombinant vector comprising the DNA according to item 8.

Item 10. A transformant obtained by transforming a host with the recombinant vector according to item 9.

Item 11. A method for producing the polypeptide according to any one of items 1 to 7, comprising the step of culturing the transformant according to item 10.

Item 12. A carrier for binding immunoglobulin comprising the polypeptide according to any one of items 1 to 7 immobilized on an insoluble carrier.

Item 13. A method for separating an immunoglobulin or a fragment thereof, comprising separating an immunoglobulin or a polypeptide containing an Fc region thereof, using the carrier for binding immunoglobulin according to item 12.

Item 14. The method according to item 13, wherein the immunoglobulin or the polypeptide containing an Fc region thereof is bound to the carrier for binding immunoglobulin, and then the immunoglobulin or the polypeptide containing an Fc region thereof is eluted at pH 3 to 5.

Advantageous Effects of Invention

The polypeptide of the present invention has binding capacity for an immunoglobulin or a polypeptide containing an Fc region thereof, and can retain its binding capacity for the immunoglobulin or the polypeptide containing an Fc region thereof even when exposed to alkaline conditions. Thus, even after repeated elution and washing with an alkaline solution, a decrease in the binding capacity for the immunoglobulin or the polypeptide containing an Fc region thereof can be inhibited. The polypeptide of the present invention also allows an immunoglobulin or a fragment thereof that has been bound to be efficiently eluted under weakly acidic (pH: about 3 to 5) mild conditions. Thus, denaturation of the immunoglobulin or the polypeptide containing an Fc region thereof to be separated can be inhibited.

Brief Description of Drawings

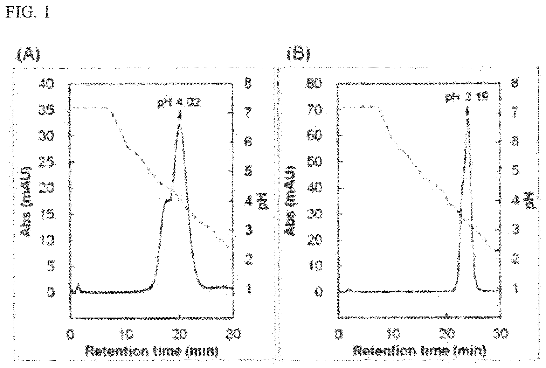

Fig. 1 shows the measured results of IgG elution profiles with a pH gradient, using the multidomain polypeptides of Example 56 (PN-621 variant) and Comparative Example 50 (PN-621); Fig. 1(A) shows the results for Example 56 (PN-621 variant), and Fig. 1(B) shows the results for Comparative Example 50 (PN-621).

Description of Embodiments

The present invention will be hereinafter described in detail. In sections other than SEQUENCE LISTING, the twenty amino acid residues in amino acid sequences may be shown as one-letter abbreviations. That is, G represents glycine (Gly), A represents alanine (Ala), V represents valine (Val), L represents leucine (Leu), I represents isoleucine (Ile), F represents phenylalanine (Phe), Y represents tyrosine (Tyr), W represents tryptophan (Trp), S represents serine (Ser), T represents threonine (Thr), C represents cysteine (Cys), M represents methionine (Met), D represents aspartic acid (Asp), E represents glutamic acid (Glu), N represents asparagine (Asn), Q represents glutamine (Gln), K represents lysine (Lys), R represents arginine (Arg), H represents histidine (His), and P represents proline (Pro).

As used herein, designations such as "Q9V" and "Q9V/S41V" refer to amino acid substitutions. For example, "Q9V" means that glutamine at position 9 from the N-terminus in a particular amino acid sequence is substituted with valine. For example, "Q9V/S41V" means that glutamine at position 9 from the N-terminus in a particular amino acid sequence is substituted with valine, and serine at position 41 from the N-terminus is substituted with valine.

As used herein, the term "amino acid with a hydrophobic side chain" includes alanine, valine, leucine, isoleucine, methionine, proline, phenylalanine, and tryptophan. As used herein, the term "amino acid with a hydrophobic aliphatic side chain" includes alanine, valine, leucine, isoleucine, and methionine.

1. Polypeptide

Embodiments of the polypeptide of the present invention include a polypeptide comprising at least one immunoglobulin-binding domain as set forth in (A):

(A) an immunoglobulin-binding domain comprising an amino acid sequence having a modification meeting the following conditions (i) to (iv) introduced into an amino acid sequence as set forth in any of SEQ ID NOS: 1 to 15:

(i) serine at position 41 is substituted with an amino acid with a hydrophobic side chain, tyrosine, or histidine;
(ii) glutamine at position 9 is unsubstituted or substituted with an amino acid with a hydrophobic aliphatic side chain or histidine;
(iii) glutamic acid or glutamine at position 15 is unsubstituted or substituted with alanine, histidine, tyrosine, or leucine; and
(iv) glutamic acid or alanine at position 24 is unsubstituted, or substituted with glutamine, histidine, or alanine in the case of SEQ ID NOS: 1 to 12, or substituted with glutamine or histidine in the case of SEQ ID NOS: 13 to 15.

The amino acid sequences as set forth in SEQ ID NOS: 1 to 15 are the amino acid sequences of the wild-type immunoglobulin-binding domains (A, B, C, D, and E) of Protein A derived from *Staphylococcus aureus* and variants thereof.

Specifically, SEQ ID NO: 1 is the wild-type C domain, SEQ ID NOS: 2 and 3 are variants of the C domain, SEQ ID NO: 4 is the wild-type A domain, SEQ ID NOS: 5 and 6 are variants of the A domain, SEQ ID NO: 7 is the wild-type B domain, SEQ ID NOS: 8 and 9 are variants of the B domain, SEQ ID NO: 10 is the wild-type D domain, SEQ ID NOS: 11 and 12 are variants of the D domain, SEQ ID NO: 13 is the wild-type E domain, and SEQ ID NOS: 14 and 15 are variants of the E domain.

The amino acid sequence as set forth in SEQ ID NO: 2 is an amino acid sequence having lysine at position 4 substituted with alanine, lysine at position 7 substituted with threonine, lysine at position 35 substituted with arginine, valine at position 40 substituted with lysine, glutamic acid at position 43 substituted with lysine, alanine at position 46 substituted with lysine, and aspartic acid at position 53 substituted with lysine in the amino acid sequence of the wild-type C domain (SEQ ID NO: 1). The introduction of lysines at positions 40, 43, 46, and 53 in the amino acid sequence as set forth in SEQ ID NO: 2 renders the sequence after position 40 lysine-rich, which facilitates immobilization on the carrier.

The amino acid sequence as set forth in SEQ ID NO: 3 is an amino acid sequence having lysine at position 4 substituted with alanine, lysine at position 7 substituted with threonine, lysine at position 35 substituted with arginine, lysine at position 42 substituted with alanine, lysine at position 49 substituted with arginine, lysine at position 50 substituted with arginine, and lysine at position 58 substituted with arginine in the amino acid sequence of the wild-type C domain (SEQ ID NO: 1).

The amino acid sequence as set forth in SEQ ID NO: 5 is an amino acid sequence having asparagine at position 4 substituted with alanine, lysine at position 7 substituted with threonine, lysine at position 35 substituted with arginine, glutamine at position 40 substituted with lysine, asparagine at position 43 substituted with lysine, serine at position 46 substituted with lysine, and glutamic acid at position 53 substituted with lysine in the amino acid sequence of the wild-type A domain (SEQ ID NO: 4). The introduction of lysines at positions 40, 43, 46, and 53 in the amino acid sequence as set forth in SEQ ID NO: 5 renders the sequence after position 40 lysine-rich, which facilitates immobilization on the carrier.

The amino acid sequence as set forth in SEQ ID NO: 6 is an amino acid sequence having asparagine at position 4 substituted with alanine, lysine at position 7 substituted with threonine, lysine at position 35 substituted with arginine, lysine at position 49 substituted with arginine, lysine at position 50 substituted with arginine, and lysine at position 58 substituted with arginine in the amino acid sequence of the wild-type A domain (SEQ ID NO: 4).

The amino acid sequence as set forth in SEQ ID NO: 8 is an amino acid sequence having lysine at position 4 substituted with alanine, lysine at position 7 substituted with threonine, lysine at position 35 substituted with arginine, glutamine at position 40 substituted with lysine, asparagine at position 43 substituted with lysine, alanine at position 46 substituted with lysine, and aspartic acid at position 53 substituted with lysine in the amino acid sequence of the wild-type B domain (SEQ ID NO: 7). The introduction of lysines at positions 40, 43, 46, and 53 in the amino acid sequence as set forth in SEQ ID NO: 8 renders the sequence after position 40 lysine-rich, which facilitates immobilization on the carrier.

The amino acid sequence as set forth in SEQ ID NO: 9 is an amino acid sequence having lysine at position 4 substituted with alanine, lysine at position 7 substituted with threonine, lysine at position 35 substituted with arginine, lysine at position 49 substituted with arginine, lysine at position 50 substituted with arginine, and lysine at position 58 substituted with arginine in the amino acid sequence of the wild-type B domain (SEQ ID NO: 7).

The amino acid sequence as set forth in SEQ ID NO: 11 is an amino acid sequence having asparagine at position 4 substituted with alanine, lysine at position 7 substituted with threonine, lysine at position 35 substituted with arginine, glutamine at position 40 substituted with lysine, asparagine at position 43 substituted with lysine, glycine at position 46 substituted with lysine, and glutamic acid at position 53 substituted with lysine in the amino acid sequence of the wild-type D domain (SEQ ID NO: 10). The introduction of lysines at positions 40, 43, 46, and 53 in the amino acid sequence as set forth in SEQ ID NO: 11 renders the sequence after position 40 lysine-rich, which facilitates immobilization on the carrier.

The amino acid sequence as set forth in SEQ ID NO: 12 is an amino acid sequence having asparagine at position 4 substituted with alanine, lysine at position 7 substituted with threonine, lysine at position 35 substituted with arginine, threonine at position 42 substituted with alanine, lysine at position 49 substituted with arginine, lysine at position 50 substituted with arginine, and lysine at position 58 substituted with arginine in the amino acid sequence of the wild-type D domain (SEQ ID NO: 10).

The amino acid sequence as set forth in SEQ ID NO: 14 is an amino acid sequence having glutamine at position 4 substituted with alanine, glutamic acid at position 7 substituted with threonine, lysine at position 35 substituted with arginine, glutamine at position 40 substituted with lysine, asparagine at position 43 substituted with lysine, glycine at position 46 substituted with lysine, and aspartic acid at position 53 substituted with lysine in the amino acid sequence of the wild-type E domain (SEQ ID NO: 13). The introduction of lysines at positions 40, 43, 46, and 53 in the amino acid sequence as set forth in SEQ ID NO: 14 renders the sequence after position 40 lysine-rich, which facilitates immobilization on the carrier.

The amino acid sequence as set forth in SEQ ID NO: 15 is an amino acid sequence having glutamine at position 4 substituted with alanine, glutamic acid at position 7 substituted with threonine, lysine at position 35 substituted with arginine, glutamine at position 49 substituted with arginine, lysine at position 50 substituted with arginine, and lysine at position 58 substituted with arginine in the amino acid sequence of the wild-type E domain (SEQ ID NO: 13).

The immunoglobulin-binding domains of Protein A include three α-helices, i.e., the first α-helix, the second α-helix, and the third α-helix, from the N-terminus. The serine at position 41 in each of the amino acid sequences as set forth in SEQ ID NOS: 1 to 15 is conserved in all of the immunoglobulin-binding domains (A to E); it is present in the third α-helix of the three α-helices of the immunoglobulin-binding domain, and forms a linkage with the second α-helix while also interacting with the first α-helix. Substitution of the serine at position 41 with an amino acid with a hydrophobic side chain, tyrosine, or histidine can increase hydrophobic interactions between amino acids within the immunoglobulin-binding domain, and improve the alkaline stability. Moreover, the serine at position 41 is located behind the α-helix structure of the glutamine at position 9, the glutamine at position 10, and the asparagine at position 11 that form immunoglobulin-binding sites in the first α-helix. Substitution of the serine at position 41 with an amino acid with a hydrophobic side chain, tyrosine, or histidine is believed to affect the IgG-binding structure formed by the above-mentioned amino acids.

The amino acid to substitute for the serine at position 41 in each of the amino acid sequences as set forth in SEQ ID NOS: 1 to 15 may be any of an amino acid with a hydrophobic side chain, tyrosine, and histidine, preferably alanine, valine, leucine, phenylalanine, tyrosine, or histidine.

The glutamine at position 9 in each of the amino acid sequences as set forth in SEQ ID NOS: 1 to 15 is one of the IgG-binding sites, and contributes to binding to an IgG by forming a hydrogen bond with the serine at position 254 of the IgG. The glutamine at position 9 in each of the amino acid sequences as set forth in SEQ ID NOS: 1 to 15 may be unsubstituted or substituted with an amino acid with a hydrophobic aliphatic side chain or histidine. If the glutamine at position 9 in each of the amino acid sequences as set forth in SEQ ID NOS: 1 to 15 is substituted with an amino acid with a hydrophobic aliphatic side chain or histidine, the elution properties in a weakly acidic range can be improved while further improving the alkaline stability. While not wishing to be construed in a limited manner, it is assumed that because the amino acids at positions 41 and 9 are in spatially close positions in each of the amino acid sequences as set forth in SEQ ID NOS: 1 to 15, substitution of the glutamine at position 9 in each of the amino acid sequences as set forth in SEQ ID NOS: 1 to 15 with an amino acid with a hydrophobic aliphatic side chain or histidine results in interactions of the amino acid with a hydrophobic side chain, tyrosine, or histidine present at position 41 with the amino acid with a hydrophobic aliphatic side chain or histidine present at position 9, which can reduce the mutual binding with an IgG to thereby reduce the avidity in a weakly acidic range, while further improving alkaline stability.

The amino acid to substitute for the glutamine at position 9 in each of the amino acid sequences as set forth in SEQ ID NOS: 1 to 15 is preferably alanine, valine, leucine, isoleucine, or histidine, and more preferably alanine, valine, leucine, or histidine.

The glutamic acid or glutamine at position 15 in each of the amino acid sequences as set forth in SEQ ID NOS: 1 to 15 (glutamic acid in the case of SEQ ID NOS: 1 to 12, and glutamine in the case of SEQ ID NOS: 13 to 15) may be unsubstituted or substituted with alanine, histidine, tyrosine or leucine. The glutamic acid at position 24 in each of the amino acid sequences as set forth in SEQ ID NOS: 1 to 12 may be unsubstituted or substituted with glutamine, histidine, or alanine. The alanine at position 24 in each of the amino acid sequences as set forth in SEQ ID NOS: 13 to 15 may be unsubstituted or substituted with glutamine or histidine. In particular, when the glutamic acid or glutamine at position 15 is substituted with alanine or histidine, and the glutamic acid or alanine at position 24 is substituted with glutamine in each of the amino acid sequences as set forth in SEQ ID NOS: 1 to 15, the alkaline stability and the elution properties in a weakly acidic range can be further improved.

Preferred examples of the immunoglobulin-binding domains as set forth in (A) include those having the following amino acid sequences as set forth in (1) to (15):

(1) an amino acid sequence having positions 9, 15, and 24 unsubstituted, and position 41 substituted with valine, phenylalanine, tyrosine, or histidine in each of the amino acid sequences as set forth in SEQ ID NOS: 1 to 15;
(2) an amino acid sequence having positions 15 and 24 unsubstituted, position 9 substituted with leucine, and position 41 substituted with valine, leucine, phenylalanine, alanine, tyrosine, or histidine in each of the amino acid sequences as set forth in SEQ ID NOS: 1 to 15;
(3) an amino acid sequence having positions 15 and 24 unsubstituted, position 9 substituted with valine, and position 41 substituted with leucine, valine, phenylalanine, tyrosine, or histidine in each of the amino acid sequences as set forth in SEQ ID NOS: 1 to 15;
(4) an amino acid sequence having positions 15 and 24 unsubstituted, position 9 substituted with histidine, and position 41 substituted with valine in each of the amino acid sequences as set forth in SEQ ID NOS: 1 to 15;

(5) an amino acid sequence having positions 15 and 24 unsubstituted, position 9 substituted with alanine, and position 41 substituted with phenylalanine, valine, tyrosine, or histidine in each of the amino acid sequences as set forth in SEQ ID NOS: 1 to 15;

(6) an amino acid sequence having positions 15 and 24 unsubstituted, position 9 substituted with isoleucine, and the serine at position 41 substituted with valine or alanine in each of the amino acid sequences as set forth in SEQ ID NOS: 1 to 15;

(7) an amino acid sequence having position 24 unsubstituted, position 9 substituted with valine, position 15 substituted with alanine, and position 41 substituted with valine in each of the amino acid sequences as set forth in SEQ ID NOS: 1 to 15;

(8) an amino acid sequence having position 24 unsubstituted, position 9 substituted with leucine or valine, position 15 substituted with histidine, and position 41 substituted with valine in each of the amino acid sequences as set forth in SEQ ID NOS: 1 to 15;

(9) an amino acid sequence having position 24 unsubstituted, position 9 substituted with valine, position 15 substituted with tyrosine, and position 41 substituted with valine in each of the amino acid sequences as set forth in SEQ ID NOS: 1 to 15;

(10) an amino acid sequence having position 24 unsubstituted, position 9 unsubstituted or substituted with leucine, position 15 substituted with leucine, and position 41 substituted with valine in each of the amino acid sequences as set forth in SEQ ID NOS: 1 to 15;

(10) an amino acid sequence having position 9 unsubstituted, position 15 substituted with alanine or histidine, position 24 substituted with glutamine, and position 41 substituted with valine or histidine in each of the amino acid sequences as set forth in SEQ ID NOS: 1 to 15;

(11) an amino acid sequence having position 9 substituted with leucine, position 15 substituted with histidine, position 24 substituted with glutamine, and position 41 substituted with valine in each of the amino acid sequences as set forth in SEQ ID NOS: 1 to 15;

(12) an amino acid sequence having position 9 substituted with leucine, position 15 substituted with histidine or leucine, position 24 substituted with glutamine, and position 41 substituted with valine in each of the amino acid sequences as set forth in SEQ ID NOS: 1 to 15;

(13) an amino acid sequence having position 15 unsubstituted, position 9 substituted with leucine, position 24 substituted with histidine, and position 41 substituted with valine in each of the amino acid sequences as set forth in SEQ ID NOS: 1 to 15;

(14) an amino acid sequence having position 9 substituted with leucine, position 15 substituted with histidine or leucine, position 24 substituted with histidine, and position 41 substituted with valine in each of the amino acid sequences as set forth in SEQ ID NOS: 1 to 15; and

(15) an amino acid sequence having position 9 substituted with valine, position 15 substituted with histidine or leucine, position 24 substituted with alanine, and position 41 substituted with valine in each of the amino acid sequences as set forth in SEQ ID NOS: 1 to 15.

Moreover, embodiments of the polypeptide of the present invention also include a polypeptide comprising at least one immunoglobulin-binding domain corresponding to the following immunoglobulin-binding domain as set forth in any of (B) and (C):

(B) an immunoglobulin-binding domain having a modification meeting the conditions (i) to (iv) introduced into an amino acid sequence as set forth in any of SEQ ID NOS: 1 to 15, wherein one or a few amino acids at sites without the modification are substituted, added, inserted, and/or deleted, and wherein the immunoglobulin-binding domain has equivalent or higher alkaline stability and higher IgG elution capacity in a weakly acidic range, as compared to a polypeptide consisting of the corresponding unmodified amino acid sequence; and

(C) an immunoglobulin-binding domain having a modification meeting the conditions (i) to (iv) introduced into an amino acid sequence as set forth in any of SEQ ID NOS: 1 to 15, wherein sequence identity of sites without the modification with respect to the corresponding unmodified amino acid sequence is 80% or more, and wherein the immunoglobulin-binding domain has equivalent or higher alkaline stability and higher IgG elution capacity in a weakly acidic range, as compared to a polypeptide consisting of the corresponding unmodified amino acid sequence.

The immunoglobulin-binding domains as set forth in (B) and (C) are variants of the immunoglobulin-binding domain as set forth in (A). The forms of the amino acid substitutions to be introduced at positions 9, 15, 24, and 41 in the amino acid sequences as set forth in SEQ ID NOS: 1 to 15, preferred amino acid substitution sites, and the like are the same as described above for the immunoglobulin-binding domain as set forth in (A).

The term "amino acids at sites without the modification" in the immunoglobulin-binding domain as set forth in (B) refers to amino acids other than the amino acid(s) substituted by the modification meeting the conditions (i) to (iv) in any of the amino acid sequences as set forth in SEQ ID NOS: 1 to 15. For example, when the modification meeting the conditions (i) to (iv) results in an amino acid substitution at position 41 only and no amino acid substitutions at positions 9, 15, and 24, the amino acids other than position 41 correspond to the "amino acids at sites without the modification". Alternatively, for example, when the modification meeting the conditions (i) to (iv) results in amino acid substitutions at positions 41 and 9 and no amino acid substitutions at positions 15 and 24, the amino acids other than positions 41 and 9 correspond to the "amino acids at sites without the modification". Alternatively, for example, when the modification meeting the conditions (i) to (iv) results in amino acid substitutions at positions 41, 15, and 24 and no amino acid substitution at position 9, the amino acids other than positions 41, 15, and 24 correspond to the "amino acids at sites without the modification".

The amino acid modification(s) to be introduced in the immunoglobulin-binding domain as set forth in (B) may include only one modification (for example, substitution) from substitution, addition, insertion, and deletion, or may include two or more modifications (for example, substitution and insertion). In the immunoglobulin-binding domain as set forth in (B), the number of the amino acids to be modified may be one, or several or a few, and may, for example, be 1 to 13, preferably 1 to 6, 1 to 5, 1 to 4, 1 to 3, 1 or 2, or 1.

The term "corresponding unmodified amino acid sequence" in the immunoglobulin-binding domain as set forth in (C) refers to the amino acid sequence used as the basis; for example, it refers to the amino acid sequence as set forth in SEQ ID NO: 1 when a modification is made to the amino acid sequence as set forth in SEQ ID NO: 1. The term "sequence identity of sites without the modification" in the immunoglobulin-binding domain as set forth in (C) refers to the sequence identity calculated by comparison in terms of amino acid sequences obtained by extracting the amino acids other than those substituted by the modification meeting the conditions (i) to (iv). For example, when the modification meeting the conditions (i) to (iv) results in an amino acid substitution at position 41 only and no amino acid substitutions at positions 9, 15, and 24, "sequence identity of sites without the modification" corresponds to the sequence identity calculated by comparison in terms of amino acid sequences obtained by extracting the amino acids other than position 41 (amino acid sequence excluding position 41). Alternatively, for example, when the modification meeting the conditions (i) to (iv) results in amino acid substitutions at positions 41 and 9 and no amino acid substitutions at positions 15 and 24, "sequence identity of sites without the modification" corresponds to the sequence identity calculated by comparison in terms of amino acid sequences obtained by extracting the amino acids other than positions 41 and 9 (amino acid sequence excluding positions 41 and 9). Alternatively, for example, when the modification meeting the conditions (i) to (iv) results in amino acid substitutions at positions 41, 15, and 24 and no amino acid substitution at position 9, "sequence identity of sites without the modification" corresponds to the sequence identity calculated by comparison in terms of amino acid sequences obtained by extracting the amino acids other than positions 41, 15, and 24 (amino acid sequence excluding positions 41, 15, and 24). The term "sequence identity" in the immunoglobulin-binding domain as set forth in (C) represents a value of amino acid sequence identity obtained using bl2seq program (Tatiana A. Tatsusova, Thomas L. Madden, FEMS Microbiol. Lett., Vol. 174, p 247-250, 1999) in BLAST PACKAGE [sgi32 bit edition, Version 2.0.12; available from National Center for Biotechnology Information (NCBI)]. The parameters may be set as follows: Gap insertion Cost value: 11, Gap extension Cost value: 1.

The sequence identity in the immunoglobulin-binding domain as set forth in (C) is not limited as long as it is 80% or more, but is preferably 85% or more, more preferably 90% or more, and still more preferably 95% or more.

Embodiments of the immunoglobulin-binding domains as set forth in (B) and (C) also include an immunoglobulin-binding domain having at least the asparagine at position 3 substituted with alanine, valine, or aspartic acid in each of the amino acid sequences as set forth in SEQ ID NOS: 1 to 12. Furthermore, embodiments of the immunoglobulin-binding domains as set forth in (B) and (C) also include an immunoglobulin-binding domain having at least the alanine at position 3 substituted with valine or aspartic acid in each of the amino acid sequences as set forth in SEQ ID NOS: 13 to 15.

Embodiments of the immunoglobulin-binding domains as set forth in (B) and (C) also include an immunoglobulin-binding domain having at least the alanine, glutamine, asparagine, or lysine at position 4 substituted with valine, histidine, arginine, or isoleucine in each of the amino acid sequences as set forth in SEQ ID NOS: 1 to 15.

Embodiments of the immunoglobulin-binding domains as set forth in (B) and (C) also include an immunoglobulin-binding domain having at least the asparagine at position 6 substituted with alanine, valine, glutamine, or aspartic acid in each of the amino acid sequences as set forth in SEQ ID NOS: 1 to 12. Furthermore, embodiments of the immunoglobulin-binding domains as set forth in (B) and (C) also include an immunoglobulin-binding domain having at least the aspartic acid at position 6 substituted with alanine, glutamine, or valine in each of the amino acid sequences as set forth in SEQ ID NOS: 13 to 15.

Embodiments of the immunoglobulin-binding domains as set forth in (B) and (C) also include an immunoglobulin-binding domain having at least the threonine or lysine at position 7 substituted with glutamic acid, histidine, arginine, or valine in each of the amino acid sequences as set forth in SEQ ID NOS: 1 to 12, 14, and 15. Furthermore, embodiments of the immunoglobulin-binding domains as set forth in (B) and (C) also include an immunoglobulin-binding domain having at least the glutamic acid at position 7 substituted with histidine, arginine, or valine in the amino acid sequence as set forth in SEQ ID NO: 13.

Embodiments of the immunoglobulin-binding domains as set forth in (B) and (C) also include an immunoglobulin-binding domain having at least the asparagine or serine at position 11 substituted with alanine, valine, histidine, glutamine, or arginine in each of the amino acid sequences as set forth in SEQ ID NOS: 1 to 15.

Specific examples of the immunoglobulin-binding domains as set forth in (B) and (C) include an immunoglobulin-binding domain having, in addition to the modification meeting the conditions (i) to (iv), at least the following amino acid substitutions in each of the amino acid sequences as set forth in SEQ ID NOS: 1 to 15:

(i) position 11 is substituted with alanine, glutamine, histidine or arginine;

(ii) position 7 is substituted with glutamic acid in the case of SEQ ID NOS: 1 to 12, 14, and 15 (position 7 is unsubstituted in the case of SEQ ID NO: 13);

(iii) position 7 is substituted with glutamic acid in the case of SEQ ID NOS: 1 to 12, 14, and 15 (position 7 is unsubstituted in the case of SEQ ID NO: 13), and position 11 is substituted with alanine or valine;

(iv) position 4 is substituted with isoleucine, and position 7 is substituted with arginine or glutamic acid in the case of SEQ ID NOS: 1 to 12, 14, and 15, or position 7 is unsubstituted or substituted with arginine in the case of SEQ ID NO: 13;

(v) position 4 is substituted with isoleucine, position 7 is substituted with arginine or glutamic acid in the case of SEQ ID NOS: 1 to 12, 14, and 15, or position 7 is unsubstituted or substituted with arginine in the case of SEQ ID NO: 13, and position 11 is substituted with alanine;

(vi) position 4 is substituted with isoleucine or valine, and position 7 is substituted with glutamic acid in the case of SEQ ID NOS: 1 to 12, 14, and 15 (position 7 is unsubstituted in the case of SEQ ID NO: 13); and

(vii) position 3 is substituted with aspartic acid, position 6 is substituted with aspartic acid in the case of SEQ ID NOS: 1 to 12 (position 6 is unsubstituted in the case of SEQ ID NOS: 13 to 15), position 7 is substituted with valine or glutamic acid in the case of SEQ ID NOS: 1 to 12, 14, and 15, or position 7 is unsubstituted or substituted with valine in the case of SEQ ID NO: 13, and position 11 is substituted with alanine.

The lysines substituting for positions 40, 43, 46, 49, 50, 53, and 58 in the amino acid sequence as set forth in SEQ ID NO: 1 serve to facilitate immobilization on the carrier, and thus, preferred embodiments of the immunoglobulin-binding domains as set forth in (B) and (C) include immunoglobulin-binding domains in which no mutations (substitutions and/or deletions) have been made at four or more, preferably six or more, more preferably all of, the lysines at positions 40, 43, 46, 49, 50, 53, and 58.

The phrase "equivalent or higher alkaline stability and higher IgG elution capacity in a weakly acidic range, as compared to a polypeptide consisting of the corresponding unmodified amino acid sequence" in the immunoglobulin-binding domains as set forth in (B) and (C) means that the immunoglobulin-binding domain has avidity for an IgG, has a residual activity after alkali treatment as measured under measurement conditions 1 shown below that is equivalent or higher than a residual activity after alkali treatment of the polypeptide consisting of the corresponding unmodified amino acid sequence (any of the amino acid sequences of SEQ ID NOS: 1 to 15 used as the basis) as measured under the same conditions, and has an elution ratio under weakly acidic conditions as measured under measurement conditions 2 shown below that is higher than an elution ratio under weakly acidic conditions of the polypeptide consisting of the corresponding unmodified amino acid sequence as measured under the same conditions. Specifically, when the residual activity after alkali treatment of the immunoglobulin-binding domain as measured under the measurement conditions 1 is at least 1.1 times the residual activity after alkali treatment of the polypeptide consisting of the corresponding unmodified amino acid sequence as measured under the same conditions, and the elution ratio under weakly acidic conditions of the immunoglobulin-binding domain as measured under the measurement conditions 2 is at least 1.2 times the elution ratio under weakly acidic conditions of the polypeptide consisting of the corresponding unmodified amino acid sequence as measured under the same conditions, the immunoglobulin-binding domain can be defined as having "equivalent or higher alkaline stability and higher IgG elution capacity in a weakly acidic range, as compared to a polypeptide consisting of the corresponding unmodified amino acid sequence". The residual activity after alkali treatment of the immunoglobulin-binding domains as set forth in (B) and (C) as measured under the measurement conditions 1 is preferably at least 1.14 times, more preferably 1.14 to 1.21 times, and still more preferably 1.15 to 1.21 times the residual activity after alkali treatment of the polypeptide consisting of the corresponding unmodified amino acid sequence as measured under the same conditions. The elution ratio under weakly acidic conditions of the immunoglobulin-binding domains as set forth in (B) and (C) as measured under the measurement conditions 2 is preferably at least 1.3 times, more preferably 1.3 to 1.52 times, and still more preferably 1.4 to 1.55 times the elution ratio under weakly acidic conditions of the polypeptide consisting of the corresponding unmodified amino acid sequence as measured under the same conditions.

<Measurement Conditions 1 (Measurement of Residual Activity after Alkali Treatment)>

The polypeptide immobilized on an agarose gel carrier is washed with an aqueous solution of 0.1 M NaOH three times and replaced with the same alkaline solution, and then stored warm at 25°C for 68 hours (alkali treatment). Next, the polypeptide is washed with PBS, and then the binding amount of human IgG (mg/ml gel) is measured. Similarly, for the polypeptide immobilized on an agarose gel carrier that has not been subjected to the alkali treatment, the binding amount of human IgG (mg/ml gel) is measured. Using the binding amount of human IgG bound to the polypeptide that has not been subjected to the alkali treatment as 100%, the ratio of the binding amount of human IgG bound to the polypeptide after the alkali treatment is calculated as "residual activity (%) after alkali treatment".

<Measurement Conditions 2 (Measurement of Elution Ratio under Weakly Acidic Conditions)>

Human IgG is bound to the polypeptide immobilized on an agarose gel carrier. Next, the immobilized polypeptide is washed with PBS, and then the human IgG bound to the immobilized polypeptide is eluted using 0.1 M citric acid buffer (pH 4.0). Subsequently, residual human IgG bound to the immobilized polypeptide is eluted using 0.1 M glycine-hydrochloric acid buffer (pH 2.8). The amount of human IgG eluted at pH 4.0 and the amount of human IgG eluted at pH 2.8 are measured. Using the total amount of the amount of human IgG eluted at pH 4.0 and the amount of human IgG eluted at pH 2.8 as 100%, the ratio of the amount of human IgG eluted at pH 4.0 is calculated as the elution ratio (%) under weakly acidic conditions.

The polypeptide of the present invention may be a single domain polypeptide having one immunoglobulin-binding domain, or may be a multidomain polypeptide in which two or more immunoglobulin-binding domains are linked. When the polypeptide of the present invention is a multidomain polypeptide, the polypeptide has the advantage of having an increased binding capacity for an immunoglobulin or a polypeptide containing an Fc region thereof.

When the polypeptide of the present invention is a single domain polypeptide, the polypeptide may contain one of the immunoglobulin-binding domains as set forth in (A) to (C).

Of the immunoglobulin-binding domains as set forth in (A) to (C), immunoglobulin-binding domains containing the amino acid sequences as set forth in SEQ ID NOS: 2, 5, 8, 11, and 14 as bases (also denoted as "lysine-rich domains", hereinafter) have a binding ability to the carrier, and therefore, when these immunoglobulin-binding domains are used as single domain polypeptides, they do not require addition of an amino acid sequence with a binding ability to the carrier; however, an amino acid sequence with a binding ability to the carrier may be added to further improve the binding ability to the carrier.

Of the immunoglobulin-binding domains as set forth in (A) to (C), when immunoglobulin-binding domains containing the amino acid sequences as set forth in SEQ ID NOS: 1, 3, 4, 6, 7, 9, 10, 12, 13, and 15 as bases (also denoted as "non-lysine-rich domains", hereinafter) are used as single domain polypeptides, an amino acid sequence with a binding ability to the carrier is preferably added to impart a binding ability to the carrier.

When the polypeptide of the present invention is a multidomain polypeptide, the polypeptide may contain at least one domain of the immunoglobulin-binding domains as set forth in (A) to (C), or may contain two or more domains of the immunoglobulin-binding domains as set forth in (A) to (C)); alternatively, the polypeptide may contain one or more domains of the immunoglobulin-binding domains as set forth in (A) to (C), and one or more of immunoglobulin-binding domains constituting Protein A and immunoglobulin-binding domains constituting Protein L. When the polypeptide of the present invention is a multidomain polypeptide, the polypeptide is preferably one in which all of the immunoglobulin-binding domains constituting the polypeptide are composed of any of the immunoglobulin-binding domains as set forth in (A) to (C), from the viewpoint of imparting further improved alkaline stability and antibody elution properties in a weakly acidic range.

When the polypeptide of the present invention is a multidomain polypeptide, the total number of linked immunoglobulin-binding domains may be 2 or more, preferably 2 to 10, and more preferably 2 to 6.

When the polypeptide of the present invention is a multidomain polypeptide, the individual immunoglobulin-binding domains constituting the polypeptide may be linked to one another directly between the C-terminus of one domain and the N-terminus of another, or may be linked to one another via 1 to 40, preferably 1 to 10 amino acid residues.

The polypeptide of the present invention in the form of a multidomain polypeptide preferably has a structure in which one or more non-lysine-rich domains and one or more lysine-rich domains are linked; more preferably has a structure in which one or more non-lysine-rich domains and one lysine-rich domain are linked; still more preferably has a structure in which two to ten non-lysine-rich domains and one lysine-rich domain are linked; and particularly has a structure in which three to five non-lysine-rich domains are linked, and one lysine-rich domain is linked to the C- or N-terminus (preferably C-terminus). When one lysine-rich domain is thus incorporated into a multidomain polypeptide, the multidomain polypeptide can be imparted with a binding ability to the carrier. Thus, the multidomain polypeptide containing a lysine-rich domain does not require addition of an amino acid sequence with a binding ability to the carrier. However, an amino acid sequence with a binding ability to the carrier may be added to this multidomain polypeptide to further improve the binding ability to the carrier.

When a multidomain polypeptide contains non-lysine-rich domains and does not contain any lysine-rich domain, an amino acid sequence with a binding ability to the carrier is preferably added to impart a binding ability to the carrier.

An exemplary amino acid sequence of the "amino acid sequence with a binding ability to the carrier" is an amino acid sequence in which 1 to 15, preferably 3 to 10, more preferably 4 to 8 lysines, are linked. This linkage sequence may contain, in addition to the lysines, an amino acid other than lysine. When the amino acid sequence with a binding ability to the carrier is added, it may be added to either the Nor C-terminus, preferably the C-terminus.

The polypeptide of the present invention may also have a peptide tag, a polypeptide with another function, or the like added at the N- or C-terminus, in order to improve the expression of the polypeptide, or impart the ease of purification, for example. The number of the amino acids to be added to the N-terminus and/or C-terminus of the polypeptide of the present invention for these purposes is, for example, 1 to 400, preferably 1 to 100, and more preferably 1 to 30, although not limited thereto.

## 2. DNA

DNA encoding the polypeptide of the present invention (hereinafter sometimes denoted as the "DNA of the present invention") may be obtained by, for example, acquiring DNA encoding an immunoglobulin-binding domain of interest by PCR or the like, using DNA encoding Protein A derived from *Staphylococcus aureus* as a template, and introducing mutations into the DNA to introduce desired amino acid substitutions or the like. The DNA of the present invention may also be artificially synthesized using a gene synthesis method.

The DNA encoding Protein A derived from *Staphylococcus aureus* is known to have the nucleotide sequence as set forth in SEQ ID NO: 16, for example, and can be isolated from *Staphylococcus aureus* by a standard method using PCR. The DNA encoding wild-type Protein A can also be artificially synthesized using a gene synthesis method.

Methods for introducing specific mutations into specific sites of a nucleotide sequence are known, and, for example, site-directed mutagenesis for DNA may be used. Specific examples of methods for converting bases in DNA include using commercial kits.

The nucleotide sequence of the DNA having mutations introduced in the nucleotide sequence can be identified using a DNA sequencer. Once the nucleotide sequence is determined, the DNA encoding the polypeptide can then be obtained by chemical synthesis, PCR using a cloned probe as a template, or hybridization using a DNA fragment having the nucleotide sequence as a probe.

Moreover, a mutant of the DNA encoding the peptide, which has an equivalent function to that of the unmutated DNA, can be synthesized by site-directed mutagenesis, for example. The introduction of a mutation into the DNA encoding the peptide can be accomplished using known methods such as the Kunkel method, the gapped duplex method, and the megaprimer PCR method.

In the DNA of the present invention, the frequency of codon usage is preferably optimized for a host. For example, when *Escherichia coli* is used as a host, DNA with a frequency of codon usage optimized for *Escherichia coli* is preferred.

## 3. Recombinant Vector

A recombinant vector comprising the DNA encoding the polypeptide of the present invention (hereinafter sometimes denoted as the "recombinant vector of the present invention") may be obtained by inserting the DNA of the present invention into an expression vector.

The recombinant vector of the present invention contains a regulatory factor such as a promoter operably linked to the DNA of the present invention. A representative example of the regulatory factor is a promoter; however, the recombinant vector of the present invention may further contain an enhancer or a transcription factor such as a CCAAT box, a TATA box, or an SPI site, as required. The phrase "operably linked" refers to the state in which the DNA of the present invention and any of various regulatory factors such as a promoter or an enhancer for regulating the DNA of the present invention are linked with each other in an operable manner in host cells.

The expression vector is preferably an expression vector constructed for genetic recombination from a phage, a plasmid, or a virus that can replicate autonomously in a host. Such expression vectors are known, and examples of commercially available expression vectors include pQE vectors (Qiagen), pDR540 and pRIT2T (GE Healthcare Bio-Sciences KK), and pET vectors (Merck Ltd.). The expression vector to be used may be selected to make an appropriate combination with host cells; for example, when *Escherichia coli* is used as host cells, preferred examples of combinations include the combination of a pET vector and *Escherichia coli* strain BL21 (DE3) and the combination of pDR540 vector and *Escherichia coli* strain JM109.

### 4. Transformant

A transformant is obtained by transforming a host with the recombinant vector of the present invention (hereinafter sometimes denoted as the "transformant of the present invention").

The host to be used for producing the transformant is not limited as long as the recombinant vector is stable, and can replicate autonomously and express the phenotype of a foreign gene. Examples of such hosts include bacteria such as the genus *Escherichia* including *Escherichia coli,* the genus *Bacillus* including *Bacillus subtilis,* and the genus *Pseudomonas* including *Pseudomonas putida;* and yeasts. Other examples of hosts include animal cells, insect cells, and plants. Among the above, *Escherichia coli* is particularly preferred.

The transformant of the present invention may be obtained by introducing the recombinant vector of the present invention into a host. The conditions under which the recombinant vector is introduced into the host may be determined as appropriate, in accordance with the type of the host, for example. When the host is a bacterium, methods include, for example, electroporation and a method using competent cells obtained by calcium ion treatment. When the host is a yeast, methods include, for example, electroporation, the spheroplast method, and the lithium acetate method. When the host is an animal cell, methods include, for example, electroporation, the calcium phosphate method, and lipofection. When the host is an insect cell, methods include, for example, the calcium phosphate method, lipofection, and electroporation. When the host is a plant, methods include, for example, electroporation, the *Agrobacterium* method, the particle gun method, and the PEG method.

### 5. Production of Polypeptide

The polypeptide of the present invention can be produced by culturing the above-described transformant.

The conditions for culturing the transformant may be determined as appropriate, in consideration of the nutrition physiological properties of the host; for example, liquid culture is preferred. For industrial production, aeration-agitation culture is preferred.

The transformant of the present invention is cultured, and the resulting culture is subjected to a method such as centrifugation to collect the culture supernatant or cells. When the polypeptide of the present invention is accumulated in cells, the cells may be subjected to sonication, a mechanical method such as French press, or a treatment with a lytic enzyme such as lysozyme, and may then be solubilized, as required, with an enzyme such as protease or a surfactant such as sodium dodecyl sulfate (SDS) to obtain water-soluble fractions containing the polypeptide of the present invention.

Alternatively, when an expression vector and a host are selected appropriately, the expressed polypeptide of the present invention can be secreted in the culture.

The culture or water-soluble fractions containing the polypeptide of the present invention obtained as described above may be directly subjected to a purification treatment, or may be subjected to a purification treatment after concentrating the polypeptide of the present invention in the culture or water-soluble fractions.

The concentration may be accomplished by, for example, vacuum concentration, membrane concentration, salting-out, or fractional precipitation using a hydrophilic organic solvent (for example, methanol, ethanol, or acetone).

The purification treatment for the polypeptide of the present invention may be accomplished by using methods such as, for example, gel filtration, hydrophobic chromatography, ion-exchange chromatography, and affinity chromatography in an appropriate combination.

The polypeptide of the present invention thus purified may be formed into a powder, as required, by lyophilization, vacuum drying, spray drying, or the like.

### 6. Carrier for Binding Immunoglobulin

The polypeptide of the present invention is immobilized on an insoluble carrier and used as a carrier for binding immunoglobulin to facilitate collection or purification of immunoglobulins. Examples of the insoluble carrier to be used to immobilize the polypeptide of the present invention include, although not limited to, naturally occurring polymer materials, such as chitosan, dextran, cellulose, and agarose; synthetic organic materials, such as vinyl alcohol, polyimide, and methacrylate; and inorganic materials, such as glass and silica.

While the shape of the insoluble carrier is not limited, the insoluble carrier may be in the form of any of a hollow fiber membrane, a monolith, or beads, for example. Among these shapes, beads are preferred because they typically have a relatively large surface area per volume, and are suitable for use in producing an affinity carrier having a high immunoglobulin binding capacity.

The polypeptide of the present invention may be immobilized on an insoluble carrier by, for example, the coupling reaction of amino groups, carboxyl groups, or thiol groups in the polypeptide of the present invention with the insoluble carrier. Specifically, the immobilization may be accomplished by, for example, activating an insoluble carrier by reacting it with a coupling agent such as cyanogen bromide, epichlorohydrin, N-hydroxysuccinimide, tosyl chloride, tresyl chloride, carbodiimide, glutaraldehyde, or hydrazine, or introducing reactive functional groups such as carboxyl groups or thiol groups into the carrier, followed by the coupling reaction between the polypeptide of the present invention and the insoluble carrier. Such coupling reactions are well-known in the art (for example, Janson, J.-C., [Protein purification], 3rd edition, pages 221-258, ISBN 978-0-471-74661-4), and may be performed in accordance with a conventional method.

Alternatively, when the polypeptide of the present invention is immobilized on an insoluble carrier via amino groups in the polypeptide, it is preferred to use a carrier having reactive functional groups (such as tresyl groups, epoxy groups, carboxyl groups, or formyl groups) that can react with amino groups to form covalent bonds. Such insoluble carriers are commercially available, for example, TOYOPEARL AF-Tresyl-650, TOYOPEARL AF-Epoxy-650, TOYOPEARL AF-Carboxy-650, and TOYOPEARL AF-Formyl-650 (all from Tosoh Corporation); NHS-activated Sepharose, Cyanogen bromide-activated Sepharose, and Epoxy-activated Sepharose (all from GE Healthcare Bio-Sciences KK); Profinity Epoxide (Bio-Rad Inc.); Glyoxal-Agarose (from Agarose Beads Technologies); and Cellufine Formyl (JNC Corporation), and these commercially available products may be used.

Alternatively, the polypeptide of the present invention may be immobilized on an insoluble carrier by adding a condensation or cross-linking reagent, such as carbodiimide or glutaraldehyde, into a system in which the polypeptide of the present invention and the insoluble carrier co-exist.

7. Method for Separating Immunoglobulin or Fragment Thereof

The polypeptide of the present invention, which binds to Fc regions of immunoglobulins, can be used to separate immunoglobulins such as IgG, IgM, and IgA, as well as polypeptides containing Fc regions thereof (such as Fc-fusion proteins).

To separate an immunoglobulin or a polypeptide containing an Fc region thereof using the polypeptide of the present invention, an insoluble carrier having the polypeptide of the present invention immobilized thereon may be used. Specifically, the separation of an immunoglobulin using an insoluble carrier having the polypeptide of the present invention immobilized thereon may be accomplished by affinity column chromatography.

The separation of an immunoglobulin by affinity column chromatography may be accomplished by passing a solution containing an immunoglobulin or a polypeptide containing an Fc region thereof through a column packed with an insoluble carrier having the polypeptide of the present invention immobilized thereon, which allows the immunoglobulin or the polypeptide containing an Fc region thereof to be bound to the polypeptide of the present invention; washing the inside of the column, as required; and passing the eluate adjusted to an appropriate pH into the column to elute the immunoglobulin or the polypeptide containing an Fc region thereof. The solution containing an immunoglobulin or a polypeptide containing an Fc region thereof may have a pH of 6.5 or more, preferably 6.5 to 8.0. While the eluate may have any pH at or below a weakly acidic level, specifically pH 5 or less, the pH of the eluate is preferably 3 to 5, more preferably 3.4 to 4.5, and still more preferably 3.6 to 4.5, in order to inhibit the separated immunoglobulin or fragment thereof from being exposed to strongly acidic conditions.

Examples

The present invention will be hereinafter specifically described with examples, although the present invention should not be interpreted as being limited to the following examples.

Text Example 1: Production of Single Domain Polypeptides and Evaluation (1)

1. Production of Single Domain Polypeptides

Single domain polypeptides were produced by modifying the C domain of Protein A. The method of production was specifically as follows.

<Production of PN-32 Expression Plasmid>

A polypeptide consisting of the amino acid sequence as set forth in SEQ ID NO: 2 was designated as PN-32. An expression plasmid for PN-32 was produced using the following method.

Double-stranded DNA of a nucleotide sequence including a recognition sequence of restriction enzyme NdeI at the site of a translation initiation codon, the sequence encoding the amino acid sequence of SEQ ID NO: 2, a translation stop codon, and a recognition sequence of restriction enzyme BamHI in this order was prepared using synthetic oligonucleotides. A fragment obtained by cleaving both ends of the synthetic oligonucleotides with restriction enzymes NdeI and BamHI was incorporated by a ligation reaction into pET9a plasmid, which was similarly cleaved with these two restriction enzymes, and then the plasmid was incorporated into *Escherichia coli* DH-5α competent cells for transformation. The *Escherichia coli* cells were cultured in the presence of kanamycin, and the expression plasmid for PN-32 (pET9a•PN-32) was purified.

The nucleic acid sequence of pET9a•PN-32 thus obtained was analyzed using a CEQ 8000 DNA sequencer (Beckman Coulter, Inc.) to confirm that the sequence was as designed.

<Production of PN-32 Variant Expression Plasmids>

Expression plasmids for PN-32 variants, each having an amino acid substitution as shown in Table 1 introduced into the amino acid sequence as set forth in SEQ ID NO: 2, were produced using the following method.

Double-stranded DNA encoding a variant of interest was prepared by PCR using pET9a•PN-32 as a template, and using an oligonucleotide DNA designed and synthesized to have a single amino acid substitution of interest, as a primer. The DNA was cleaved with restriction enzymes Ndel and BamHI as in the preparation of pET9a•PN-32, and incorporated into pET9a plasmid by a ligation reaction to give an expression plasmid for the PN-32 variant having the single amino acid substitution. Additionally, expression plasmids for PN-32 variants each having two amino acid substitutions were created by carrying out the same procedure as described above, using expression plasmids for variants each having a single amino acid substitution as templates.

The nucleic acid sequences of the expression plasmids for the PN-32 variants thus obtained were analyzed with a CEQ 8000 DNA sequencer (Beckman Coulter, Inc.) to confirm that the sequences were as designed.

<Production of PN-32 and Variants Thereof>

*Escherichia coli* BL21 (DE3) competent cells (Merck Ltd.) were transformed with the expression plasmids for PN-32 and the variants thereof obtained above to give strains expressing PN-32 and the variants thereof.

The *Escherichia coli* strains expressing PN-32 and the variants thereof were seed-cultured for 12 hours in LB medium containing 25 mg/L of kanamycin and 2.0% glucose. The resulting seed cultures were inoculated into 2x TY medium containing 25 mg/L of kanamycin and 0.8% glucose, and cultured at 37°C for 16 hours to express PN-32 and the variants thereof of interest. Then, the cultures were centrifuged to collect *Escherichia coli* cells. Next, the collected *Escherichia coli* cells were suspended in 50 mM sodium phosphate buffer (pH 6.5), the suspensions were sonicated to disrupt the *Escherichia coli* cells, and the cells were further centrifuged to collect PN-32 and the variants thereof in the supernatants. The resulting supernatants as cell extracts were subjected to sodium dodecyl sulfate-15% polyacrylamide gel electrophoresis (SDS-PAGE), which confirmed the production of PN-32 and the variants thereof as intended.

The cell extracts of the *Escherichia coli* strains expressing PN-32 and the variants thereof were adjusted to pH 6.0 and then applied to a cation exchanger SP-Sepharose Fast Flow (GE Healthcare KK) column. The column was washed with 20 mM phosphate buffer (pH 6.0), and then proteins were eluted from the column with a linear concentration gradient of 0.5 M NaCl. SDS-PAGE of the eluates confirmed that PN-32 and the variants thereof were eluted between 0.1 to 0.2 M NaCl. Next, the eluates containing PN-32 and the variants thereof were adjusted to pH 9 and then added to an anion exchanger GigaCap Q (Tosoh Corporation) column. The column was washed with 20 mM phosphate buffer (pH 7.8), and then PN-32 and the variants thereof were separated with a linear concentration gradient of 0.3 M NaCl. The eluates were subjected to SDS-PAGE to determine the purity, and the results confirmed that PN-32 and the variants thereof were each purified as a single band in the position of the theoretical molecular weight.

2. Immobilization of Single Domain Polypeptides on Gel Support

PN-32 and the variants thereof thus purified were immobilized on a formyl-activated agarose gel carrier at a concentration of 10 mg/mL gel, in accordance with a conventional method. The reaction solutions after the immobilization were collected, and the immobilization efficiency was measured. As a result, all of PN-32 and the variants thereof showed an immobilization efficiency of 90% or more.

3. Measurement of Immunoglobulin Avidity

The gel carriers having PN-32 and the variants thereof immobilized thereon were washed with PBS. Then, PBS (pH 7.5) containing 40 mg/mL of human polyclonal IgG (available from Japan Blood Products Organization) was added thereto, and the mixtures were shaken for 1 hour. Then, the gel carriers were washed with PBS (pH 7.5). Next, the human IgG bound to the gel carriers was eluted from the gel carriers, using 0.1 M glycine hydrochloride buffer (pH 2.8). Each eluate was subjected to measurement of absorbance at 280 nm with a spectrophotometer, and the amount of IgG bound (binding amount of IgG) was determined based on a specific absorption coefficient of 13.8 (1 g$^{-1}$ cm$^{-1}$).

4. Measurement of Residual Activity after Alkali Treatment

The gel carriers having PN-32 and the variants thereof immobilized thereon were washed with PBS and then further washed with an aqueous solution of 0.1 M NaOH three times and replaced with the same alkaline solution, and then stored at 25°C for 68 hours (alkali treatment). Next, the gel carriers were washed with PBS three times, and then the immunoglobulin avidity was measured under the same conditions as described above. Using the binding amount of IgG in each eluate before the alkali treatment as 100%, the ratio of the binding amount of IgG remaining after the alkali treatment was determined as "residual activity (%) after alkali treatment".

5. Measurement of Elution Ratio under Weakly Acidic Conditions

The gel carriers having PN-32 and the variants thereof immobilized thereon were washed with PBS. Then, PBS (pH 7.5) containing 40 mg/mL of human polyclonal IgG (available from Japan Blood Products Organization) was added thereto, and the mixtures were shaken for 1 hour. Then, the gel carriers were washed with PBS (pH 7.5). Next, the human IgG bound to the gel carriers was eluted using 0.1 M citric acid buffer (pH 4.0). Subsequently, residual human IgG bound to the gel carriers was eluted using 0.1 M glycine-hydrochloric acid buffer (pH 2.8). The eluate eluted at pH 4.0 and the eluate eluted at pH 2.8 were subjected to measurement of absorbance at 280 nm with a spectrophotometer, and the amount of IgG contained in each of the eluates was determined based on a specific absorption coefficient of 13.8 (1 $g^{-1}$ $cm^{-1}$). Using the total amount of the amount of human IgG in the eluate eluted at pH 4.0 and the amount of human IgG in the eluate eluted at pH 2.8 as 100%, the ratio of the amount of human IgG in the eluate eluted at pH 4.0 was calculated as the "elution ratio (%) under weakly acidic conditions".

6. Results

The results are shown in Table 1. The results revealed that in the variants having the serine at position 41 substituted with an amino acid with a hydrophobic side chain (valine or phenylalanine), histidine, or tyrosine in PN-32 (SEQ ID NO: 2), the residual activity after alkali treatment was improved to at least 1.1 times that of PN-32, and the elution ratio under weakly acidic conditions was improved to at least 1.2 times that of PN-32. Only position 41 proved to be the modification site with a single mutation that achieved these two effects simultaneously.

In the variants having the glutamine at position 9 substituted with an amino acid with a hydrophobic aliphatic side chain (alanine, valine, leucine, or isoleucine), and the serine at position 41 substituted with an amino acid with a hydrophobic side chain (alanine, valine, leucine, or phenylalanine), tyrosine, or histidine, the residual activity after alkali treatment was confirmed to be even higher than that of the variants with a single mutation at position 41, and the elution ratio under weakly acidic conditions was confirmed to be dramatically improved over PN-32. When the variants having the serine at position 41 substituted with alanine or leucine further had the glutamine at position 9 substituted with an amino acid with a hydrophobic aliphatic side chain or histidine, the residual activity after alkali treatment was found to be high, and the elution ratio under weakly acidic conditions was found to be dramatically improved over the single-mutation variants. It should be noted that in the variants having the glutamine at position 9 substituted with phenylalanine, and serine at position 41 substituted with an amino acid with a hydrophobic side chain, tyrosine, or histidine, the residual activity after alkali treatment was not higher than that of the variants having a substitution at position 41 only.

[Table 1]

| Single Domain Polypeptides [PN-32 (SEQ ID NO: 2) and Variants Thereof] | | | | | |
|---|---|---|---|---|---|
| | | | Evaluation Results | | |
| | | Types of Mutations in SEQ ID NO: 2 | Elution Ratio (%) under Weakly Acidic Conditions The value in parentheses is a relative ratio with respect to PN-32. | Residual Activity (%) after Alkali Treatment (68 hr with 0.1 M NaOH) The value in parentheses is a relative ratio with respect to PN-32. | Binding Amount of IgG (mg/mL gel) |
| Comp. Ex. 1 | PN-32 | None | 61.4 (1.00) | 71.9 (1.00) | 76.3 |
| Comp. Ex. 2 | PN-32 Variant | Q9L | 85.8 (1.40) | 77.7 (1.08) | 62.3 |
| Comp. Ex. 3 | PN-32 Variant | Q9A | 68.6 (1.12) | 79.5 (1.11) | 73.7 |

(continued)

| Single Domain Polypeptides [PN-32 (SEQ ID NO: 2) and Variants Thereof] | | | | | |
|---|---|---|---|---|---|
| | | | Evaluation Results | | |
| | | Types of Mutations in SEQ ID NO: 2 | Elution Ratio (%) under Weakly Acidic Conditions The value in parentheses is a relative ratio with respect to PN-32. | Residual Activity (%) after Alkali Treatment (68 hr with 0.1 M NaOH) The value in parentheses is a relative ratio with respect to PN-32. | Binding Amount of IgG (mg/mL gel) |
| Comp. Ex. 4 | PN-32 Variant | Q9V | 79.2 (1.29) | 78.3 (1.09) | 68.5 |
| Comp. Ex. 5 | PN-32 Variant | Q9F | 90.4 (1.47) | 74.9 (1.04) | 60.3 |
| Comp. Ex. 6 | PN-32 Variant | Q9I | 87.0 (1.42) | 74.5 (1.04) | 63.5 |
| Comp. Ex. 7 | PN-32 Variant | Q9H | 96.5 (1.57) | 52.3 (0.73) | 67.1 |
| Comp. Ex. 8 | PN-32 Variant | Q9E | 60.9 (0.99) | 61.3 (0.85) | 61.6 |
| Comp. Ex. 9 | PN-32 Variant | Q9T | 82.3 (1.34) | 60.6 (0.84) | 66.8 |
| Comp. Ex. 10 | PN-32 Variant | Q9Y | 91.4 (1.49) | 64.8 (0.90) | 57.3 |
| Comp. Ex. 11 | PN-32 Variant | N11H | 57.0 (0.93) | 83.9 (1.17) | 70.2 |
| Comp. Ex. 12 | PN-32 Variant | N11A | 60.1 (0.98) | 82.7 (1.15) | 80.5 |
| Comp. Ex. 13 | PN-32 Variant | N11S | 60.7 (0.99) | 68.9 (0.96) | 82.0 |
| Comp. Ex. 14 | PN-32 Variant | N11T | 61.9 (1.01) | 72.9 (1.01) | 77.9 |
| Comp. Ex. 15 | PN-32 Variant | N11V | 59.9 (0.98) | 80.2 (1.12) | 76.5 |
| Comp. Ex. 16 | PN-32 Variant | N11E | 65.1 (1.06) | 86.4 (1.20) | 72.8 |
| Comp. Ex. 17 | PN-32 Variant | N11L | 68.3 (1.11) | 88.1 (1.23) | 70.0 |
| Comp. Ex. 18 | PN-32 Variant | N11R | 63.1 (1.03) | 87.0 (1.21) | 74.1 |
| Comp. Ex. 19 | PN-32 Variant | N11Y | 67.5 (1.10) | 83.6 (1.16) | 72.8 |
| Comp. Ex. 20 | PN-32 Variant | N11F | 68.2 (1.11) | 83.0 (1.15) | 72.0 |
| Comp. Ex. 21 | PN-32 Variant | N11D | 56.2 (0.92) | 79.0 (1.10) | 66.9 |
| Comp. Ex. 22 | PN-32 Variant | N11Q | 65.0 (1.06) | 86.7 (1.21) | 73.0 |

(continued)

| Single Domain Polypeptides [PN-32 (SEQ ID NO: 2) and Variants Thereof] | | | | | |
|---|---|---|---|---|---|
| | | | Evaluation Results | | |
| | | Types of Mutations in SEQ ID NO: 2 | Elution Ratio (%) under Weakly Acidic Conditions The value in parentheses is a relative ratio with respect to PN-32. | Residual Activity (%) after Alkali Treatment (68 hr with 0.1 M NaOH) The value in parentheses is a relative ratio with respect to PN-32. | Binding Amount of IgG (mg/mL gel) |
| Comp. Ex. 23 | PN-32 Variant | A12H | 95.8 (1.56) | 51.2 (0.71) | 53.7 |
| Comp. Ex. 24 | PN-32 Variant | A12V | 79.4 (1.29) | 71.4 (0.99) | 65.0 |
| Comp. Ex. 25 | PN-32 Variant | A12L | 95.9 (1.56) | 33.6 (0.47) | 49.0 |
| Comp. Ex. 26 | PN-32 Variant | A12E | 86.8 (1.41) | 10.7 (0.15) | 44.2 |
| Comp. Ex. 27 | PN-32 Variant | A12T | 77.4 (1.26) | 49.8 (0.69) | 68.6 |
| Comp. Ex. 28 | PN-32 Variant | A12R | 94.8 (1.54) | 28.7 (0.40) | 51.2 |
| Comp. Ex. 29 | PN-32 Variant | E15H | 69.9 (1.14) | 73.7 (1.03) | 75.4 |
| Comp. Ex. 30 | PN-32 Variant | E15A | 64.9 (1.06) | 79.0 (1.10) | 73.6 |
| Comp. Ex. 31 | PN-32 Variant | E15L | 70.0 (1.14) | 76.6 (1.07) | 76.8 |
| Comp. Ex. 32 | PN-32 Variant | EISD | 63.8 (1.04) | 50.0 (0.70) | 73.1 |
| Comp. Ex. 33 | PN-32 Variant | E15R | 67.7 (1.10) | 70.0 (0.97) | 76.9 |
| Comp. Ex. 34 | PN-32 Variant | E15Y | 76.3 (1.24) | 68.9 (0.96) | 69.2 |
| Comp. Ex. 35 | PN-32 Variant | E24H | 72.0 (1.17) | 66.1 (0.92) | 70.1 |
| Comp. Ex. 36 | PN-32 Variant | E24A | 67.9 (1.11) | 70.4 (0.98) | 75.7 |
| Comp. Ex. 37 | PN-32 Variant | E24Q | 69.1 (1.13) | 70.2 (0.98) | 72.2 |
| Comp. Ex. 38 | PN-32 Variant | E24R | 73.5 (1.20) | 62.8 (0.87) | 71.2 |
| Comp. Ex. 39 | PN-32 Variant | E24T | 68.3 (1.11) | 66.2 (0.92) | 72.1 |
| Comp. Ex. 40 | PN-32 Variant | E24V | 70.9 (1.16) | 64.6 (0.90) | 70.7 |
| Comp. Ex. 41 | PN-32 Variant | E24L | 79.8 (1.30) | 61.7 (0.86) | 66.7 |

(continued)

| | | Types of Mutations in SEQ ID NO: 2 | Elution Ratio (%) under Weakly Acidic Conditions The value in parentheses is a relative ratio with respect to PN-32. | Residual Activity (%) after Alkali Treatment (68 hr with 0.1 M NaOH) The value in parentheses is a relative ratio with respect to PN-32. | Binding Amount of IgG (mg/mL gel) |
|---|---|---|---|---|---|
| Single Domain Polypeptides [PN-32 (SEQ ID NO: 2) and Variants Thereof] | | | | | |
| | | | Evaluation Results | | |
| Comp. Ex. 42 | PN-32 Variant | E24Y | 62.9 (1.02) | 68.6 (0.95) | 72.0 |
| Comp. Ex. 43 | PN-32 Variant | S41T | 73.3 (1.19) | 68.9 (0.96) | 70.3 |
| Comp. Ex. 44 | PN-32 Variant | S41R | 93.4 (1.52) | 39.0 (0.54) | 57.3 |
| Comp. Ex. 45 | PN-32 Variant | S41Q | 90.4 (1.47) | 43.7 (0.61) | 62.7 |
| Comp. Ex. 46 | PN-32 Variant | S41E | 87.3 (1.42) | 38.2 (0.53) | 55.1 |
| Comp. Ex. 47 | PN-32 Variant | S41D | 84.8 (1.38) | 52.4 (0.73) | 57.6 |
| Comp. Ex. 48 | PN-32 Variant | S41G | 71.4 (1.16) | 54.9 (0.76) | 72.7 |
| Ex. 1 | PN-32 Variant | S41V | 79.6 (1.30) | 79.1 (1.10) | 68.6 |
| Ex. 2 | PN-32 Variant | S41F | 90.5 (1.47) | 82.0 (1.14) | 59.3 |
| Ex. 3 | PN-32 Variant | S41H | 84.0 (1.37) | 80.3 (1.12) | 65.7 |
| Ex. 4 | PN-32 Variant | S41Y | 90.5 (1.47) | 87.1 (1.21) | 58.7 |
| Ex. 5 | PN-32 Variant | Q9L/S41V | 90.8 (1.48) | 83.2 (1.16) | 62.3 |
| Ex. 6 | PN-32 Variant | Q9V/S41L | 93.7 (1.53) | 76.8 (1.07) | 60.1 |
| Ex. 7 | PN-32 Variant | Q9V/S41V | 85.9 (1.40) | 86.1 (1.20) | 66.3 |
| Ex. 8 | PN-32 Variant | Q9L/S41L | 95.2 (1.55) | 79.3 (1.10) | 55.6 |
| Ex. 9 | PN-32 Variant | Q9L/S41F | 92.4 (1.50) | 83.6 (1.16) | 60.0 |
| Ex. 10 | PN-32 Variant | Q9V/S41F | 88.5 (1.44) | 86.5 (1.20) | 61.8 |
| Ex. 11 | PN-32 Variant | Q9F/S41F | 95.7 (1.56) | 76.5 (1.06) | 46.7 |
| Ex. 12 | PN-32 Variant | Q9A/S41F | 84.8 (1.38) | 83.1 (1.16) | 63.7 |

(continued)

| Single Domain Polypeptides [PN-32 (SEQ ID NO: 2) and Variants Thereof] | | | | | |
|---|---|---|---|---|---|
| | | | Evaluation Results | | |
| | | Types of Mutations in SEQ ID NO: 2 | Elution Ratio (%) under Weakly Acidic Conditions The value in parentheses is a relative ratio with respect to PN-32. | Residual Activity (%) after Alkali Treatment (68 hr with 0.1 M NaOH) The value in parentheses is a relative ratio with respect to PN-32. | Binding Amount of IgG (mg/mL gel) |
| Ex. 13 | PN-32 Variant | Q9L/S41A | 82.9 (1.35) | 81.4 (1.13) | 66.7 |
| Ex. 14 | PN-32 Variant | Q9L/S41Y | 92.3 (1.50) | 82.6 (1.15) | 59.6 |
| Ex. 15 | PN-32 Variant | Q9V/S41Y | 87.6 (1.43) | 84.0 (1.17) | 63.4 |
| Ex. 16 | PN-32 Variant | Q9F/S41Y | 95.6 (1.56) | 72.2 (1.00) | 48.3 |
| Ex. 17 | PN-32 Variant | Q9A/S41Y | 84.7 (1.38) | 86.9 (1.21) | 62.5 |
| Ex. 18 | PN-32 Variant | Q9L/S41H | 91.4 (1.49) | 83.8 (1.17) | 57.4 |
| Ex. 19 | PN-32 Variant | Q9V/S41H | 86.1 (1.40) | 84.7 (1.18) | 60.1 |
| Ex. 20 | PN-32 Variant | Q9F/S41H | 95.2 (1.55) | 77.4 (1.08) | 49.9 |
| Ex. 21 | PN-32 Variant | Q9A/S41H | 84.3 (1.37) | 83.4 (1.16) | 64.6 |
| Ex. 22 | PN-32 Variant | Q9F/S41V | 95.5 (1.56) | 75.0 (1.04) | 50.0 |
| Ex. 23 | PN-32 Variant | Q9A/S41V | 82.7 (1.35) | 82.6 (1.15) | 63.5 |
| Ex. 24 | PN-32 Variant | Q9I/S41V | 91.6 (1.49) | 85.4 (1.89) | 63.8 |
| Ex. 25 | PN-32 Variant | Q9I/S41A | 90.3 (1.47) | 87.0 (1.21) | 62.1 |

Text Example 2: Production of Single Domain Polypeptides and Evaluation (2)

1. Production of Single Domain Polypeptides

Expression plasmids for PN-32 variants, each having amino acid substitutions as shown in Table 2 introduced into PN32 (SEQ ID NO: 2), were produced as in Test Example 1.

The nucleic acid sequences of the expression plasmids for the PN-32 variants thus obtained were analyzed with a CEQ 8000 DNA sequencer (Beckman Coulter, Inc.) to confirm that the sequences were as designed.

<Production of PN-32 Variants>

*Escherichia coli* BL21 (DE3) competent cells (Merck Ltd.) were transformed with the expression plasmids for the PN-32 variants obtained above to give strains expressing PN-32 and the variants thereof.

The *Escherichia coli* strains expressing the PN-32 variants were seed-cultured for 12 hours in LB medium containing 25 mg/L of kanamycin and 2.0% glucose. The resulting seed cultures were inoculated into 2x TY medium containing 25 mg/L of kanamycin and 0.8% glucose, and cultured at 37°C for 16 hours to express PN-32 and the variants thereof of interest. Then, the cultures were centrifuged to collect *Escherichia coli* cells. Next, the collected *Escherichia coli* cells were suspended in 50 mM sodium phosphate buffer (pH 6.5), the suspensions were sonicated to disrupt the *Escherichia coli* cells, and the cells were further centrifuged to collect the PN-32 variants in the supernatants. The resulting supernatants as cell extracts were subjected to sodium dodecyl sulfate-15% polyacrylamide gel electrophoresis (SDS-PAGE), which confirmed the production of the PN-32 variants as intended.

The cell extracts of the *Escherichia coli* strains expressing the PN-32 variants were adjusted to pH 6.0 and then applied to a cation exchanger SP-Sepharose Fast Flow (GE Healthcare KK) column. The column was washed with 20 mM phosphate buffer (pH 6.0), and then proteins were eluted from the column with a linear concentration gradient of 0.5 M NaCl. SDS-PAGE of the eluates confirmed that the PN-32 variants were eluted between 0.1 to 0.2 M NaCl. Next, the eluates containing the PN-32 variants thereof were adjusted to pH 9 and then added to an anion exchanger GigaCap Q (Tosoh Corporation) column. The column was washed with 20 mM phosphate buffer (pH 7.8), and then the PN-32 variants were separated with a linear concentration gradient of 0.3 M NaCl. The eluates were subjected to SDS-PAGE to determine the purity, and the results confirmed that the PN-32 variants were each purified as a single band in the position of the theoretical molecular weight.

## 2. Immobilization of Single Domain Polypeptides on Gel Support

The PN-32 variants thus purified were immobilized on a formyl-activated agarose gel carrier at a concentration of 10 mg/mL gel, in accordance with a conventional method. The reaction solutions after the immobilization were collected, and the immobilization efficiency was measured. As a result, all of the PN-32 variants showed an immobilization efficiency of 90% or more.

## 3. Measurement of Immunoglobulin Avidity

Using the gel carriers having PN-32 and the variants thereof immobilized thereon, the binding amount of IgG was determined under the same conditions as in Text Example 1.

## 4. Measurement of Residual Activity after Alkali Treatment

The gel carriers having the PN-32 variants immobilized thereon were washed with PBS and then further washed with an aqueous solution of 0.5 M NaOH three times and replaced with the same alkaline solution, and then stored at 25°C for 17 hours (alkali treatment). Next, the gel carriers were washed with PBS three times, and then the immunoglobulin avidity was measured under the same conditions as described above. Using the binding amount of IgG in each eluate before the alkali treatment as 100%, the ratio of the binding amount of IgG remaining after the alkali treatment was determined as "residual activity (%) after alkali treatment".

## 5. Measurement of Elution Ratio under Weakly Acidic Conditions

Using the gel carriers having PN-32 and the variants thereof immobilized thereon, the elution ratio under weakly acidic conditions was determined under the same conditions as in Text Example 1.

## 6. Results

The results are shown in Table 2. The results confirmed that similarly in the variants having position 9 substituted with an amino acid with a hydrophobic aliphatic side chain, position 41 substituted with an amino acid with a hydrophobic side chain, and position 11 substituted with alanine in PN-32 (SEQ ID NO: 2), the residual activity after alkali treatment was dramatically improved to at least 1.49 times that of PN-32, and the elution ratio under weakly acidic conditions was dramatically improved to 1.27 times that of PN-32. Moreover, in the variant having position 9 substituted with an amino acid with a hydrophobic aliphatic side chain, position 41 substituted with an amino acid with a hydrophobic side chain, position 15 substituted with alanine, and position 11 substituted with arginine in PN-32 (SEQ ID NO: 2), the alkaline stability was improved and simultaneously, the elution ratio under weakly acidic conditions was further improved. Furthermore, similarly in the variants having position 9 unsubstituted, position 15 substituted with alanine or histidine, position 24 substituted with glutamine, and position 41 substituted with an amino acid with a hydrophobic side chain, the residual activity after alkali treatment was confirmed to be dramatically improved to at least 1.40 times that of PN-32, and the elution ratio under weakly acidic conditions dramatically improved to 1.40 times that of PN-32.

[Table 2]

| Single Domain Polypeptides [PN-32 (SEQ ID NO: 2) and Variants ThereofJ | | | | | |
|---|---|---|---|---|---|
| | | | Evaluation Results | | |
| | | Types of Mutations in SEQ ID NO: 2 | Elution Ratio (%) under Weakly Acidic Conditions The value in parentheses is a relative ratio with respect to PN-32. | Residual Activity (%) after Alkali Treatment (17 hr with 0.5 M NaOH) The value in parentheses is a relative ratio with respect to PN-32. | Binding Amount of IgG (mg/mL gel) |
| Comp. Ex. 1 | PN-32 | None | 61.4 (1.00) | 56.1(1.00) | 76.3 |
| Ex. 26 | PN-32 Variant | Q9A/N11A/S41V | 77.7 (1.27) | 84.8 (1.51) | 65.5 |
| Ex. 27 | PN-32 Variant | Q9V/N11A/S41V | 80.3 (1.31) | 83.8 (1.49) | 66.9 |
| Ex. 28 | PN-32 Variant | Q9V/N11A/E15A/S41V | 82.5 (1.34) | 83.6 (1.49) | 65.3 |
| Ex. 29 | PN-32 Variant | Q9V/N11R/E15A/S41V | 90.3 (1.47) | 83.3 (1.48) | 58.2 |
| Ex. 30 | PN-32 Variant | Q9I/N11A/S41V | 84.6 (1.38) | 83.2 (1.48) | 64.9 |
| Ex. 31 | PN-32 Variant | Q9I/N11A/S41A | 83.4 (1.36) | 86.7 (1.55) | 63.1 |
| Ex. 32 | PN-32 Variant | E15A/E24Q/S41V | 85.7 (1.40) | 80.1 (1.43) | 64.1 |
| Ex. 33 | PN-32 Variant | E15H/E24Q/S41V | 90.2 (1.47) | 78.3 (1.40) | 61.2 |

Text Example 3: Production of Single Domain Polypeptides and Evaluation (3)

1. Production of Single Domain Polypeptides

Single domain polypeptides were produced by modifying the C domain of Protein A. The method of production was specifically as follows.

<Production of PN-128 Expression Plasmid>

A polypeptide consisting of an amino acid sequence having the threonine at position 7 substituted with glutamic acid in the amino acid sequence as set forth in SEQ ID NO: 2 was designated as PN-128. An expression plasmid for PN-128 was produced as in Test Example 1.
The nucleic acid sequence of the expression plasmid for PN-128 thus obtained was analyzed using a CEQ 8000 DNA sequencer (Beckman Coulter, Inc.) to confirm that the sequence was as designed.

<Production of PN-128 Variant Expression Plasmids>

Expression plasmids for PN-128 variants, each having amino acid substitutions as shown in Table 3 introduced into the amino acid sequence as set forth in SEQ ID NO: 2, were produced as in Test Example 1.
The nucleic acid sequences of the expression plasmids for the PN-128 variants thus obtained were analyzed with a CEQ 8000 DNA sequencer (Beckman Coulter, Inc.) to confirm that the sequences were as designed.

<Production of PN-128 and Variants Thereof>

*Escherichia coli* BL21 (DE3) competent cells (Merck Ltd.) were transformed with the expression plasmids for PN-128 and the variants thereof obtained above to give strains expressing PN-32 and the variants thereof.

The *Escherichia coli* strains expressing PN-128 and the variants thereof were seed-cultured for 12 hours in LB medium containing 25 mg/L of kanamycin and 2.0% glucose. The resulting seed cultures were inoculated into 2x TY medium containing 25 mg/L of kanamycin and 0.8% glucose, and cultured at 37°C for 16 hours to express PN-128 and the variants thereof of interest. Then, the cultures were centrifuged to collect *Escherichia coli* cells. Next, the collected *Escherichia coli* cells were suspended in 50 mM sodium phosphate buffer (pH 6.5), the suspensions were sonicated to disrupt the *Escherichia coli* cells, and the cells were further centrifuged to collect PN-32 and the variants thereof in the supernatants. The resulting supernatants as cell extracts were subjected to sodium dodecyl sulfate-15% polyacrylamide gel electrophoresis (SDS-PAGE), which confirmed the production of PN-128 and the variants thereof as intended.

The cell extracts of the *Escherichia coli* strains expressing PN-128 and the variants thereof were adjusted to pH 6.0 and then applied to a cation exchanger SP-Sepharose Fast Flow (GE Healthcare KK) column. The column was washed with 20 mM phosphate buffer (pH 6.0), and then proteins were eluted from the column with a linear concentration gradient of 0.5 M NaCl. SDS-PAGE of the eluates confirmed that PN-128 and the variants thereof were eluted between 0.1 to 0.2 M NaCl. Next, the eluates containing PN-128 and the variants thereof were adjusted to pH 9 and then added to an anion exchanger GigaCap Q (Tosoh Corporation) column. The column was washed with 20 mM phosphate buffer (pH 7.8), and then PN-128 and the variants thereof were separated with a linear concentration gradient of 0.3 M NaCl. The eluates were subjected to SDS-PAGE to determine the purity, and the results confirmed that PN-128 and the variants thereof were each purified as a single band in the position of the theoretical molecular weight.

2. Immobilization of Single Domain Polypeptides on Gel Support

PN-128 and the variants thereof thus purified were immobilized on a formyl-activated agarose gel carrier at a concentration of 10 mg/mL gel, in accordance with a conventional method. The reaction solutions after the immobilization were collected, and the immobilization efficiency was measured. As a result, all of PN-128 and the variants thereof showed an immobilization efficiency of 90% or more.

3. Measurement of Immunoglobulin Avidity

Using the gel carriers having PN-128 and the variants thereof immobilized thereon, the binding amount of IgG was determined under the same conditions as in Text Example 1.

4. Measurement of Residual Activity after Alkali Treatment

Using the gel carriers having PN-128 and the variants thereof immobilized thereon, the residual activity after alkali treatment was determined under the same conditions as in Text Example 2.

5. Measurement of Elution Ratio under Weakly Acidic Conditions

Using the gel carriers having PN-128 and the variants thereof immobilized thereon, the elution ratio under weakly acidic conditions was determined under the same conditions as in Text Example 1.

6. Results

The results are shown in Table 3. The results confirmed that in the variants each having position 9 substituted with an amino acid with a hydrophobic aliphatic side chain, and position 41 substituted with an amino acid with a hydrophobic side chain or histidine, and additionally having at least one of the substitutions: (i) substitution of position 11 with an amino acid with a hydrophobic side chain, (ii) substitution of position 15 with an amino acid with a hydrophobic side chain, tyrosine or histidine, and (iii) substitution of position 24 with alanine, glutamine, or histidine, in PN-128 (amino acid sequence having position 7 substituted with glutamic acid in the amino acid sequence as set forth in SEQ ID NO: 2), the residual activity after alkali treatment was equivalent or higher than that of PN-128, and the elution ratio under weakly acidic conditions was dramatically improved over PN-128.

[Table 3]

| Single Domain Polypeptides [PN-128 and Variants Thereof] | | | | | |
|---|---|---|---|---|---|
| | | | Evaluation Results | | |
| | | Types of Mutations in SEQ ID NO: 2 | Elution Ratio (%) under Weakly Acidic Conditions The value in parentheses is a relative ratio with respect to PN-128. | Residual Activity (%) after Alkali Treatment (17 hr with 0.5 M NaOH) The value in parentheses is a relative ratio with respect to PN-128. | Binding Amount of IgG (mg/mL gel) |
| Comp. Ex. 49 | PN-128 | T7E | 58.5 (1.00) | 66.8 (1.00) | 73.5 |
| Ex. 34 | PN-128 Variant | T7E/Q9V/E24Q/ S41V | 90.6 (1.55) | 84.7 (1.27) | 60.7 |
| Ex. 35 | PN-128 Variant | T7E/Q9V/E24Q/ S41H | 88.7 (1.52) | 84.3 (1.26) | 60.6 |
| Ex. 36 | PN-128 Variant | T7E/Q9A/N11A/ S41H | 76.6 (1.31) | 85.4 (1.28) | 69.0 |
| Ex. 37 | PN-128 Variant | T7E/Q9V/N11V/ S41V | 85.5 (1.46) | 82.5 (1.24) | 63.5 |
| Ex. 38 | PN-128 Variant | T7E/Q9V/E15Y/ S41V | 88.6 (1.51) | 79.7 (1.19) | 60.6 |
| Ex. 39 | PN-128 Variant | T7E/Q9V/N11A/ S41V | 77.7 (1.33) | 87.5 (1.31) | 70.2 |
| Ex. 40 | PN-128 Variant | T7E/Q9V/N11A/ E15A/S41V | 81.4 (1.39) | 87.8 (1.31) | 69.7 |
| Ex. 41 | PN-128 Variant | T7E/Q9L/E15H/ S41V | 92.2 (1.58) | 75.0 (1.12) | 62.6 |
| Ex. 42 | PN-128 Variant | T7E/Q9L/E24H/ S41V | 94.1 (1.61) | 74.4 (1.11) | 57.2 |
| Ex. 43 | PN-128 Variant | T7E/Q9L/E15H/ E24H/S41V | 95.6 (1.63) | 73.2 (1.10) | 56.6 |
| Ex. 44 | PN-128 Variant | T7E/Q9L/E15L/ E24H/S41V | 95.8 (1.64) | 75.0 (1.12) | 55.0 |
| Ex. 45 | PN-128 Variant | T7E/Q9L/E15H/ E24Q/S41V | 95.6 (1.63) | 82.2 (1.23) | 55.5 |
| Ex. 46 | PN-128 Variant | T7E/Q9V/E15H/ E24A/S41V | 92.7 (1.58) | 74.8 (1.12) | 61.4 |
| Ex. 47 | PN-128 Variant | T7E/Q9V/E15A/ E24A/S41V | 91.5 (1.56) | 77.1 (1.15) | 59.9 |

Text Example 4: Production of Multidomain Polypeptides and Evaluation

1. Production of Multidomain Polypeptides

A multidomain polypeptide having six modified sequences of the C domain of Protein A was produced. The method of production was specifically as follows.

<Production of PN-621 Expression Plasmid>

A polypeptide consisting of an amino acid sequence in which five amino acid sequences of the amino acid sequence as set forth in SEQ ID NO: 3 (sequence a) and one amino acid sequence of the amino acid sequence as set forth in SEQ ID NO: 2 (sequence β) had been directly linked in this order from the N-terminus was designated as PN-621. The expression plasmid for PN-621 was produced using the method disclosed in Patent Literature 3.

The nucleic acid sequence of the expression plasmid for PN-621 thus obtained was analyzed using a CEQ 8000 DNA sequencer (Beckman Coulter, Inc.) to confirm that the sequence was as designed.

<Production of PN-621 Variant Expression Plasmids>

Expression plasmids for PN-621 variants in which substitutions having amino acid sequences as shown in Table 4 had been introduced were produced using the methods disclosed in Test Example 1 and Patent Literature 3.

The nucleic acid sequences of the expression plasmids for the PN-621 variants thus obtained were analyzed with a CEQ 8000 DNA sequencer (Beckman Coulter, Inc.) to confirm that the sequences were as designed.

<Production of PN-621 and Variants Thereof>

*Escherichia coli* BL21 (DE3) competent cells (Merck Ltd.) were transformed with the expression plasmids for PN-621 and the variants thereof obtained above to give strains expressing PN-621 and the variants thereof.

The *Escherichia coli* strains expressing PN-621 and the variants thereof were seed-cultured for 12 hours in LB medium containing 25 mg/L of kanamycin and 2.0% glucose. The resulting seed cultures were inoculated into 2x TY medium containing 25 mg/L of kanamycin and 0.8% glucose, and cultured at 37°C for 16 hours to express PN-621 and the variants thereof of interest. Then, the cultures were centrifuged to collect *Escherichia coli* cells. Next, the collected *Escherichia coli* cells were suspended in 50 mM sodium phosphate buffer (pH 6.5), the suspensions were sonicated to disrupt the *Escherichia coli* cells, and the cells were further centrifuged to collect PN-621 and the variants thereof in the supernatants. The resulting supernatants as cell extracts were subjected to sodium dodecyl sulfate-15% polyacrylamide gel electrophoresis (SDS-PAGE), which confirmed the production of PN-621 and the variants thereof as intended.

The cell extracts of the *Escherichia coli* strains expressing PN-621 and the variants thereof were adjusted to pH 6.0 and then applied to a cation exchanger SP-Sepharose Fast Flow (GE Healthcare KK) column. The column was washed with 20 mM phosphate buffer (pH 6.0), and then proteins were eluted from the column with a linear concentration gradient of 0.5 M NaCl. The eluates containing PN-621 and the variants thereof were adjusted to pH 8 and then added to an anion exchanger GigaCap Q (Tosoh Corporation) column. The column was washed with 20 mM phosphate buffer (pH 7.8), and then PN-621 and the variants thereof were separated with a linear concentration gradient of 0.3 M NaCl. The eluates were subjected to SDS-PAGE to determine the purity, and the results confirmed that PN-621 and the variants thereof were each purified as a single band in the position of the theoretical molecular weight.

2. Immobilization of Multidomain Polypeptides on Gel Support

PN-621 and the variants thereof thus purified were immobilized on a formyl-activated agarose gel carrier at a concentration of 10 mg/mL gel, in accordance with a conventional method. The reaction solutions after the immobilization were collected, and the immobilization efficiency was measured. As a result, all of PN-621 and the variants thereof showed an immobilization efficiency of 90% or more.

3. Measurement of Immunoglobulin Avidity

A 0.5 mL amount of each of the gel carriers having PN-621 and the variants thereof immobilized thereon was packed in a Tricorn 5/20 column (GE Healthcare KK). The column was set on a liquid chromatography system AKTA pure 25 (GE Healthcare KK) and equilibrated with PBS, and then a human polyclonal IgG (available from Japan Blood Products Organization) solution with a concentration of 3 mg/mL was passed at a flow rate with a retention time of 5 minutes. Absorbance at 280 nm of the solution passing through the column was monitored to determine the amount of added IgG at the point when the IgG concentration in the pass-through solution reached 10% of the IgG concentration in the IgG solution used. Using this value, the dynamic binding amount of IgG per mL of the gel (mg/mL gel) was calculated according to the following formula:

[Expression 1]

Dynamic binding amount of IgG (mg/mL gel) = addition amount (mg) of IgG / column capacity (mL)

Addition amount (mg) of IgG = addition amount (mL) of IgG solution × IgG concentration (mg/mL)

Addition amount of IgG solution: addition amount of the IgG solution at the point when the IgG concentration in the pass-through solution reached 10% of the IgG concentration in the IgG solution used.

4. Measurement of Residual Activity after Alkali Treatment

Using the gel carriers having PN-621 and the variants thereof immobilized thereon, the residual activity after alkali treatment was determined under the same conditions as in Text Example 2.

5. Measurement of Elution Ratio under Weakly Acidic Conditions

Using the gel carriers having PN-621 and the variants thereof immobilized thereon, the elution ratio under weakly acidic conditions was determined under the same conditions as in Text Example 1.

6. Results

The results are shown in Table 4. The results revealed that similarly in the multidomain polypeptides having a plurality of amino acid sequences each having position 41 substituted with an amino acid with a hydrophobic side chain or histidine in SEQ ID NO: 2 or 3; in the multidomain polypeptides having a plurality of amino acid sequences each having position 9 substituted with an amino acid with a hydrophobic aliphatic side chain or histidine, and position 41 substituted with an amino acid with a hydrophobic side chain or histidine in SEQ ID NO: 2 or 3; and the multidomain polypeptide having a plurality of amino acid sequences each having position 9 unsubstituted, position 15 substituted with histidine, position 24 substituted with glutamine, and position 41 substituted with histidine in SEQ ID NO: 2 or 3, the residual activity after alkali treatment and the IgG elution capacity under weakly acidic conditions were high.

[Table 4]

| Multidomain Polypeptides [PN-621 and Variants Thereof] | | | | | |
|---|---|---|---|---|---|
| | | | Evaluation Results | | |
| | | Amino Acid Sequence#1 | Elution Ratio (%) under Weakly Acidic Conditions The value in parentheses is a relative ratio with respect to PN-621. | Residual Activity (%) after Alkali Treatment (17 hr with 0.5 M NaOH) The value in parentheses is a relative ratio with respect to PN-621. | Binding Amount of IgG (mg! ml, gel) |
| Comp. Ex. 50 | PN-621 | Amino acid sequence having five sequences α and one sequence β directly linked from the N-terminus | 46.4 (1.00) | 46.5 (1.00) | 73.0 |
| Ex. 48 | PN-621 Variant | Amino acid sequence having five sequences α1 and one sequence β1 directly linked from the N-terminus | 61.9 (1.33) | 80.8 (1.78) | 71.9 |
| Ex. 49 | PN-621 Variant | Amino acid sequence having five sequences α2 and one sequence β2 directly linked from the N-terminus | 72.1 (1.55) | 84.6 (1.82) | 70.8 |
| Ex. 50 | PN-621 Variant | Amino acid sequence having six sequences α2 and six lysines directly linked from the N-terminus | 72.5 (1.56) | 85.1 (1.83) | 73.2 |

(continued)

| Multidomain Polypeptides [PN-621 and Variants Thereof] | | | | | |
|---|---|---|---|---|---|
| | | | Evaluation Results | | |
| | | Amino Acid Sequence[#1] | Elution Ratio (%) under Weakly Acidic Conditions The value in parentheses is a relative ratio with respect to PN-621. | Residual Activity (%) after Alkali Treatment (17 hr with 0.5 M NaOH) The value in parentheses is a relative ratio with respect to PN-621. | Binding Amount of IgG (mg! ml, gel) |
| Ex. 51 | PN-621 Variant | Amino acid sequence having five sequences $\alpha 3$ and one sequence $\beta 3$ directly linked from the N-terminus | 83.1 (1.79) | 73.8 (1.59) | 66.4 |
| Ex. 52 | PN-621 Variant | Amino acid sequence having five sequences $\alpha 4$ and one sequence $\beta 4$ directly linked from the N-terminus | 73.1 (1.58) | 86.1 (1.85) | 70.6 |
| Ex. 53 | PN-621 Variant | Amino acid sequence having five sequences $\alpha 5$ and one sequence $\beta 5$ directly linked from the N-terminus | 71.5 (1.54) | 82.9 (1.78) | 72.0 |
| Ex. 54 | PN-621 Variant | Amino acid sequence having five sequences $\alpha 6$ and one sequence $\beta 6$ directly linked from the N-terminus | 72.3 (1.56) | 82.7 (1.78) | 72.9 |
| Ex. 55 | PN-621 Variant | Amino acid sequence having six sequences $\alpha 6$ and six lysines directly linked from the N-terminus | 72.6 (1.56) | 83.2 (1.79) | 73.6 |
| Ex. 56 | PN-621 Variant | Amino acid sequence having five sequences $\alpha 7$ and one sequence $\beta 7$ directly linked from the N-terminus | 83.6 (1.80) | 75.1 (1.62) | 72.3 |
| Ex. 57 | PN-621 Variant | Amino acid sequence having five sequences $\alpha 8$ and one sequence $\beta 8$ directly linked from the N-terminus | 75.7 (1.63) | 86.1 (1.85) | 66.0 |
| Ex. 58 | PN-621 Variant | Amino acid sequence having five sequences $\alpha 9$ and one sequence $\beta 9$ directly linked from the N-terminus | 71.4 (1.54) | 80.6 (1.73) | 69.7 |

(continued)

| Multidomain Polypeptides [PN-621 and Variants Thereof] | | | | | |
|---|---|---|---|---|---|
| | | | Evaluation Results | | |
| | | Amino Acid Sequence[#1] | Elution Ratio (%) under Weakly Acidic Conditions The value in parentheses is a relative ratio with respect to PN-621. | Residual Activity (%) after Alkali Treatment (17 hr with 0.5 M NaOH) The value in parentheses is a relative ratio with respect to PN-621. | Binding Amount of IgG (mg! ml, gel) |
| Ex. 59 | PN-621 Variant | Amino acid sequence having five sequences α10 and one sequence β10 directly linked from the N-terminus | 82.9 (1.79) | 84.8 (1.82) | 69.0 |
| Ex. 60 | PN-621 Variant | Amino acid sequence having six sequences α11 and eight lysines directly linked from the N-tenninus | 72.0 (1.55) | 88.6 (1.91) | 72.9 |
| Ex. 61 | PN-621 Variant | Amino acid sequence having six sequences α12 and eight lysines directly linked from the N-terminus | 72.6 (1.56) | 89.9 (1.93) | 71.4 |
| Ex. 62 | PN-621 Variant | Amino acid sequence having six sequences α13 and eight lysines directly linked from the N-terminus | 83.8 (1.81) | 88.5 (1.90) | 75.1 |
| Ex. 63 | PN-621 Variant | Amino acid sequence having six sequences α14 and eight lysines directly linked from the N-terminus | 85.2 (1.84) | 87.8 (1.89) | 68.2 |

#1: Sequence α, sequence β, sequences α1 to α12, and sequences β1 to β10 shown in Table 4 represent the following sequences:

Sequence α: amino acid sequence as set forth in SEQ ID NO: 3
Sequence β: amino acid sequence as set forth in SEQ ID NO: 2
Sequence α1: amino acid sequence having the amino acid substitutions A4I/T7R/N11A/S41V introduced into the amino acid sequence as set forth in SEQ ID NO: 3
Sequence β1: amino acid sequence having the amino acid substitutions A4I/T7R/N11A/S41V introduced into the amino acid sequence as set forth in SEQ ID NO: 2
Sequence α2: amino acid sequence having the amino acid substitutions A4I/T7R/Q9V/N11A/S41V introduced into the amino acid sequence as set forth in SEQ ID NO: 3
Sequence β2: amino acid sequence having the amino acid substitutions A4I/T7R/Q9V/N11A/S41V introduced into the amino acid sequence as set forth in SEQ ID NO: 2
Sequence α3: amino acid sequence having the amino acid substitutions A4V/T7E/Q9L/E15H/S41V introduced into the amino acid sequence as set forth in SEQ ID NO: 3
Sequence β3: amino acid sequence having the amino acid substitutions A4V/T7E/Q9L/E15H/S41V introduced into the amino acid sequence as set forth in SEQ ID NO: 2

Sequence α4: amino acid sequence having the amino acid substitutions A4I/T7E/N11H/E15L/S41V introduced into the amino acid sequence as set forth in SEQ ID NO: 3

Sequence β4: amino acid sequence having the amino acid substitutions A4I/T7E/N11H/E15L/S41V introduced into the amino acid sequence as set forth in SEQ ID NO: 2

Sequence α5: amino acid sequence having the amino acid substitutions A4I/T7E/Q9A/N11A/S41V introduced into the amino acid sequence as set forth in SEQ ID NO: 3

Sequence β5: amino acid sequence having the amino acid substitutions A4I/T7E/Q9A/N11A/S41V introduced into the amino acid sequence as set forth in SEQ ID NO: 2

Sequence α6: amino acid sequence having the amino acid substitutions A41/T7E/Q9V/N11A/S41V introduced into the amino acid sequence as set forth in SEQ ID NO: 3

Sequence β6: amino acid sequence having the amino acid substitutions A4I/T7E/Q9V/N11A/S41V introduced into the amino acid sequence as set forth in SEQ ID NO: 2

Sequence α7: amino acid sequence having the amino acid substitutions A4I/T7E/Q9H/N11A/S41V introduced into the amino acid sequence as set forth in SEQ ID NO: 3

Sequence β7: amino acid sequence having the amino acid substitutions A4I/T7E/Q9H/N11A/S41V introduced into the amino acid sequence as set forth in SEQ ID NO: 2

Sequence α8: amino acid sequence having the amino acid substitutions A4I/T7R/Q9V/N11A/E15H/S41V introduced into the amino acid sequence as set forth in SEQ ID NO: 3

Sequence β8: amino acid sequence having the amino acid substitutions A4I/T7R/Q9V/N11A/E15H/S41V introduced into the amino acid sequence as set forth in SEQ ID NO: 2

Sequence α9: amino acid sequence having the amino acid substitutions A4I/T7E/Q9V/N11A/E15A/S41V introduced into the amino acid sequence as set forth in SEQ ID NO: 3

Sequence β9: amino acid sequence having the amino acid substitutions A4I/T7E/Q9V/N11A/E15A/S41V introduced into the amino acid sequence as set forth in SEQ ID NO: 2

Sequence α10: amino acid sequence having the amino acid substitutions A41/T7E/Q9L/N11H/E15L/S41V introduced into the amino acid sequence as set forth in SEQ ID NO: 3

Sequence β10: amino acid sequence having the amino acid substitutions A4I/T7E/Q9L/N11H/E15L/S41V introduced into the amino acid sequence as set forth in SEQ ID NO: 2

Sequence α11: amino acid sequence having the amino acid substitutions N3D/A4A/N6D/T7V/Q9A/N11A/S41V introduced into the amino acid sequence as set forth in SEQ ID NO: 3

Sequence α12: amino acid sequence having the amino acid substitutions N3D/A4A/N6D/T7E/Q9V/N11A/S41V introduced into the amino acid sequence as set forth in SEQ ID NO: 3

Sequence α13: amino acid sequence having the amino acid substitutions N3V/A4A/N6Q/T7V/Q9I/N11Q/S41V introduced into the amino acid sequence as set forth in SEQ ID NO: 3

Sequence α14: amino acid sequence having the amino acid substitutions N3D/A4V/N6Q/T7V/N11Q/E15H/E24Q/S41H introduced into the amino acid sequence as set forth in SEQ ID NO: 3

Test Example 5: IgG Elution Profile with pH Gradient

Human polyclonal IgG (available from Japan Blood Products Organization) was injected into a column packed with a gel carrier having the multidomain polypeptide of Example 56 (PN-621 variant) or Comparative Example 50 (PN-621) immobilized thereon and bound thereto. Next, the human IgG was eluted with a linear pH gradient, using 0.1 M citric acid buffer at pH 7.2 and 0.1 M citric acid buffer at pH 2.3, and the absorbance of the eluate at 280 nm was monitored.
The results are shown in Fig. 1. Fig. 1(A) shows the results obtained for Example 56 (PN-621 variant), and Fig. 1(B) shows the results obtained for Comparative Example 50 (PN-621). With the gel carrier having the multidomain polypeptide of Example 56 immobilized thereon, the human IgG was eluted at pH 4.02. On the other hand, with the gel carrier having the multidomain polypeptide of Comparative Example 50 (PN-621) immobilized thereon, the human IgG was eluted at pH 3.19. These results clearly indicate that the Protein A variant of the present invention allows an IgG to be purified at a higher pH value under weakly acidic conditions.

Test Example 6: Production of Wild-Type Single Domain Polypeptides and Variants Thereof, and Evaluation

1. Production of Wild-Type, Single-Mutation, and Double-Mutation Single Domain Polypeptides

Expression plasmids for wild-type single domain polypeptides of the five domains (A, B, C, D, and E) of Protein A, and for variants thereof were prepared. Specifically, expression plasmids for wild-type A domain (SEQ ID NO: 4), wild-type B domain (SEQ ID NO: 7), wild-type C domain (SEQ ID NO: 1), wild-type D domain (SEQ ID NO: 10), wild-type E domain (SEQ ID NO: 13), single-mutation variants having position 41 substituted with histidine in these wild-type domains, and double-mutation variants having position 9 substituted with leucine and position 41 substituted with histidine in these wild-type domains were produced as in Test Example 1. These wild-type domains and the variants thereof each had a sequence in which five lysines had been linked at the C-terminus to make them usable for immobilization on a gel carrier.

The nucleic acid sequences of the expression plasmids for the wild-type single domain polypeptides and the variants thereof were analyzed with a CEQ 8000 DNA sequencer (Beckman Coulter, Inc.) to confirm that the sequences were as designed.

<Production of Domain Variants>

*Escherichia coli* BL21 (DE3) competent cells (Merck Ltd.) were transformed with the expression plasmids obtained above to give strains expressing the wild-type single domain polypeptides and the variants thereof.

The expression *Escherichia coli* strains obtained above were seed-cultured for 12 hours in LB medium containing 25 mg/L of kanamycin and 2.0% glucose. The resulting seed cultures were inoculated into 2x TY medium containing 25 mg/L of kanamycin and 0.8% glucose, and cultured at 37°C for 16 hours to express the wild-type single domain polypeptides and the variants thereof of interest, and then the cultures were centrifuged to collect *Escherichia coli* cells. Next, the collected *Escherichia coli* cells were suspended in 50 mM sodium phosphate buffer (pH 6.5), the suspensions were sonicated to disrupt the *Escherichia coli* cells, and the cells were further centrifuged to collect the wild-type single domain polypeptides and the variants thereof in the supernatants. The resulting supernatants as cell extracts were subjected to sodium dodecyl sulfate-15% polyacrylamide gel electrophoresis (SDS-PAGE), which confirmed the production of the wild-type single domain polypeptides and the variants thereof as intended.

The cell extracts of the expression *Escherichia coli* strains obtained above were adjusted to pH 6.0 and then applied to a cation exchanger SP-Sepharose Fast Flow (GE Healthcare KK) column. The column was washed with 20 mM phosphate buffer (pH 6.0), and then proteins were eluted from the column with a linear concentration gradient of 0.5 M NaCl. SDS-PAGE of the eluates confirmed that the wild-type single domain polypeptides and the variants thereof were eluted between 0.1 and 0.2 M NaCl . Next, the eluates containing the wild-type single domain polypeptides and the variants thereof were adjusted to pH 9 and then added to an anion exchanger GigaCap Q (Tosoh Corporation) column. The column was washed with 20 mM phosphate buffer (pH 7.8), and then the wild-type single domain polypeptides and the variants thereof were separated with a linear concentration gradient of 0.3 M NaCl. The eluates were subjected to SDS-PAGE to determine the purity, and the results confirmed that the wild-type single domain polypeptides and the variants thereof were each purified as a single band in the position of the theoretical molecular weight.

2. Immobilization of Single Domain Polypeptides on Gel Support

The wild-type single domain polypeptides and the variants thereof thus purified were immobilized on a formyl-activated agarose gel carrier at a concentration of 10 mg/mL gel, in accordance with a conventional method. The reaction solutions after the immobilization were collected, and the immobilization efficiency was measured. As a result, all of the wild-type single domain polypeptides and the variants thereof showed an immobilization efficiency of 90% or more.

3. Measurement of Immunoglobulin Avidity

Using the gel carriers having the wild-type single domain polypeptides and the variants thereof immobilized thereon, the binding amount of IgG was determined under the same conditions as in Text Example 1.

4. Measurement of Residual Activity after Alkali Treatment

The gel carriers having the wild-type single domain polypeptides and the variants thereof immobilized thereon were washed with PBS and then further washed with an aqueous solution of 0.1 M NaOH three times and replaced with the same alkaline solution, and then stored at 25°C for 6, 17, or 68 hours (alkali treatment). Next, the gel carriers were washed with PBS three times, and then the immunoglobulin avidity was measured under the same conditions as described above. Using the binding amount of IgG in each eluate before the alkali treatment as 100%, the ratio of the binding amount of IgG remaining after the alkali treatment was determined as "residual activity (%) after alkali treatment".

5. Measurement of Elution Ratio under Weakly Acidic Conditions

Using the gel carriers having the wild-type single domain polypeptides and the variants thereof immobilized thereon, the elution ratio under weakly acidic conditions was determined under the same conditions as in Text Example 1, except that 0.1 M citric acid buffer (pH 5.0) was used for elution of the IgG.

6. Results

The results are shown in Tables 5 and 6. The results confirmed that in the single-mutation variants having position 41 substituted with histidine, and the double-mutation variants having position 9 substituted with leucine and position 41 substituted with histidine, in the five domains of Protein A, the residual activities after alkali treatment were equivalent or higher than those of the wild-types, and the elution ratios under weakly acidic conditions were also improved over the wild types.

[Table 5]

| | Relative Ratio of Elution Ratio under Weakly Acidic Conditions (Relative Value with Elution Ratio of Wild-Type Taken as 1) | | |
| --- | --- | --- | --- |
| | Wild-Type | Single-Mutation Variant (S41H) | Double-Mutation Variant (Q9L/S41H) |
| A Domain | 1.00 | 1.30 | 1.47 |
| B Domain | 1.00 | 1.21 | 1.25 |
| C Domain | 1.00 | 1.20 | 1.23 |
| D Domain | 1.00 | 1.42 | 1.54 |
| E Domain | 1.00 | 1.22 | 2.07 |

[Table 6]

| | Relative Ratio of Residual Activity After Alkali Treatment (Relative Value with Residual Activity of Wild-Type Taken as 1) | | | |
| --- | --- | --- | --- | --- |
| | Wild-Type | Single-Mutation Variant (S41H) | Double-Mutation Variant (Q9L/S41H) | Alkali Treatment Conditions |
| A Domain | 1.00 | 1.05 | 1.08 | 0.1 M NaOH, 17hr |
| B Domain | 1.00 | 1.07 | 1.16 | 0.1 M NaOH, 68hr |
| C Domain | 1.00 | 1.07 | 1.10 | 0.1 M NaOH, 68hr |
| D Domain | 1.00 | 1.00 | 1.21 | 0.1 M NaOH, 6hr |
| E Domain | 1.00 | 1.00 | 1.81 | 0.1 M NaOH, 6hr |

**Claims**

1. A polypeptide comprising at least one immunoglobulin-binding domain as set forth in any of (A) to (C):

(A) an immunoglobulin-binding domain comprising an amino acid sequence having a modification meeting the following conditions (i) to (iv) introduced into an amino acid sequence as set forth in any of SEQ ID NOS: 1 to 15:

(i) serine at position 41 is substituted with an amino acid with a hydrophobic side chain, tyrosine, or histidine;
(ii) glutamine at position 9 is unsubstituted or substituted with an amino acid with a hydrophobic aliphatic side chain or histidine;
(iii) glutamic acid or glutamine at position 15 is unsubstituted or substituted with alanine, histidine, tyrosine, or leucine; and
(iv) glutamic acid or alanine at position 24 is unsubstituted, or substituted with glutamine, histidine, or alanine in the case of SEQ ID NOS: 1 to 12, or substituted with glutamine or histidine in the case of SEQ ID NOS: 13 to 15;

(B) an immunoglobulin-binding domain having a modification meeting the conditions (i) to (iv) introduced into an amino acid sequence as set forth in any of SEQ ID NOS: 1 to 15, wherein one or a few amino acids at sites without the modification are substituted, added, inserted, and/or deleted, and wherein the immunoglobulin-binding domain has equivalent or higher alkaline stability and higher IgG elution capacity in a weakly acidic range, as compared to a polypeptide consisting of the corresponding unmodified amino acid sequence; and
(C) an immunoglobulin-binding domain having a modification meeting the conditions (i) to (iv) introduced into an amino acid sequence as set forth in any of SEQ ID NOS: 1 to 15, wherein sequence identity of sites without the modification with respect to the corresponding unmodified amino acid sequence is 80% or more, and wherein the immunoglobulin-binding domain has equivalent or higher alkaline stability and higher IgG elution capacity in a weakly acidic range, as compared to a polypeptide consisting of the corresponding unmodified amino acid sequence.

2. The polypeptide according to claim 1, which is a single domain peptide comprising one immunoglobulin-binding domain selected from the immunoglobulin-binding domains as set forth in (A) to (C).

3. The polypeptide according to claim 1, which is a multidomain peptide wherein two or more immunoglobulin-binding domains selected from the immunoglobulin-binding domains as set forth in (A) to (C) are linked.

4. The polypeptide according to any one of claims 1 to 3, wherein glutamine at position 9 is substituted with an amino acid with a hydrophobic aliphatic side chain or histidine in the amino acid sequence.

5. The polypeptide according to any one of claims 1 to 4, wherein glutamic acid or glutamine at position 15 is substituted with alanine or histidine in the amino acid sequence, and
glutamic acid at position 24 is substituted with glutamine in the case of SEQ ID NOS: 1 to 12, or alanine at position 24 is substituted with glutamine in the case of SEQ ID NOS: 13 to 15.

6. The polypeptide according to any one of claims 1 to 5, wherein serine at position 41 is substituted with alanine, valine, leucine, phenylalanine, tyrosine, or histidine in the amino acid sequence.

7. The polypeptide according to any one of claims 1 to 6, wherein glutamine at position 9 is substituted with alanine, valine, leucine, isoleucine, or histidine in the amino acid sequence.

8. DNA encoding the polypeptide according to any one of claims 1 to 7.

9. A recombinant vector comprising the DNA according to claim 8.

10. A transformant obtained by transforming a host with the recombinant vector according to claim 9.

11. A method for producing the polypeptide according to any one of claims 1 to 7, comprising the step of culturing the transformant according to claim 10.

12. A carrier for binding immunoglobulin comprising the polypeptide according to any one of claims 1 to 7 immobilized on an insoluble carrier.

13. A method for separating an immunoglobulin or a fragment thereof, comprising separating an immunoglobulin or a polypeptide containing an Fc region thereof, using the carrier for binding immunoglobulin according to claim 12.

14. The method according to claim 13, wherein the immunoglobulin or the polypeptide containing an Fc region thereof is bound to the carrier for binding immunoglobulin, and then the immunoglobulin or the polypeptide containing an Fc region thereof is eluted at pH 3 to 5.

FIG. 1

## INTERNATIONAL SEARCH REPORT

| International application No. |
| --- |
| **PCT/JP2021/047570** |

### A.    CLASSIFICATION OF SUBJECT MATTER

*C12N 15/31*(2006.01)i; *C07K 1/16*(2006.01)i; *C07K 14/31*(2006.01)i; *C07K 17/02*(2006.01)i; *C12N 1/15*(2006.01)i; *C12N 1/19*(2006.01)i; *C12N 1/21*(2006.01)i; *C12N 5/10*(2006.01)i; *C12N 15/63*(2006.01)i; *C12P 21/02*(2006.01)i
FI:    C12N15/31 ZNA; C12N15/63 Z; C07K14/31; C07K17/02; C07K1/16; C12N1/15; C12N1/19; C12N1/21; C12N5/10; C12P21/02 C

According to International Patent Classification (IPC) or to both national classification and IPC

### B.    FIELDS SEARCHED

Minimum documentation searched (classification system followed by classification symbols)

C12N15/00-15/90; C07K1/00-19/00

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

JSTPlus/JMEDPlus/JST7580 (JDreamIII); CAplus/REGISTRY/MEDLINE/EMBASE/BIOSIS (STN); UniProt/GeneSeq

### C.    DOCUMENTS CONSIDERED TO BE RELEVANT

| Category* | Citation of document, with indication, where appropriate, of the relevant passages | Relevant to claim No. |
| --- | --- | --- |
| X | PASTOR, J. J. et al. Redesign of protein domains using one-bead-one-compound combinatorial chemistry. J. Am. Chem. Soc. 2007, 129(48), pp. 14922-14932 abstract, p. 14924, left column, paragraph [0002] to p. 14930, right column, paragraph [0002], tables 1-2, fig. 10 | 1-2, 6 |
| Y | | 1-14 |
| Y | JP 2016-523959 A (GE HEALTHCARE BIO-SCIENCES AB) 12 August 2016 (2016-08-12) abstract, claims | 1-14 |
| Y | JP 2013-256479 A (NATIONAL INSTITUTE OF ADVANCED INDUSTRIAL SCIENCE& TECHNOLOGY) 26 December 2013 (2013-12-26) abstract, claims, paragraph [0107] | 1-14 |
| Y | WO 2012/165544 A1 (NATIONAL INSTITUTE OF ADVANCED INDUSTRIAL SCIENCE& TECHNOLOGY) 06 December 2012 (2012-12-06) abstract, paragraphs [0031]-[0032], [0042]-[0043], examples | 1-14 |

✓ Further documents are listed in the continuation of Box C.      ✓ See patent family annex.

| * | Special categories of cited documents: |
| --- | --- |
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
| --- | --- |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
| --- | --- |
| **18 February 2022** | **08 March 2022** |

| Name and mailing address of the ISA/JP | Authorized officer |
| --- | --- |
| **Japan Patent Office (ISA/JP)** <br> **3-4-3 Kasumigaseki, Chiyoda-ku, Tokyo 100-8915** <br> **Japan** | |
| | Telephone No. |

Form PCT/ISA/210 (second sheet) (January 2015)

## INTERNATIONAL SEARCH REPORT

| International application No. |
| --- |
| **PCT/JP2021/047570** |

**C.    DOCUMENTS CONSIDERED TO BE RELEVANT**

| Category* | Citation of document, with indication, where appropriate, of the relevant passages | Relevant to claim No. |
| --- | --- | --- |
| Y | JP 2010-156687 A (MILLIPORE CORP) 15 July 2010 (2010-07-15)<br>    claims, table 1 | 1-14 |
| P, X | CN 112210013 A (BIOPROCESSIA TECH LLC) 12 January 2021 (2021-01-12)<br>    claims, examples, fig. 5, 8 | 1-3, 6, 8-14 |
| P, Y | | 1-14 |

Form PCT/ISA/210 (second sheet) (January 2015)

## INTERNATIONAL SEARCH REPORT

| International application No. |
|---|
| **PCT/JP2021/047570** |

**Box No. I**   **Nucleotide and/or amino acid sequence(s) (Continuation of item 1.c of the first sheet)**

1. With regard to any nucleotide and/or amino acid sequence disclosed in the international application, the international search was carried out on the basis of a sequence listing:

   a. ☑ forming part of the international application as filed:

   ☑ in the form of an Annex C/ST.25 text file.

   ☐ on paper or in the form of an image file.

   b. ☐ furnished together with the international application under PCT Rule 13*ter*.1(a) for the purposes of international search only in the form of an Annex C/ST.25 text file.

   c. ☐ furnished subsequent to the international filing date for the purposes of international search only:

   ☐ in the form of an Annex C/ST.25 text file (Rule 13*ter*.1(a)).

   ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

Form PCT/ISA/210 (continuation of first sheet) (January 2015)

## INTERNATIONAL SEARCH REPORT
### Information on patent family members

| | International application No. |
|---|---|
| | **PCT/JP2021/047570** |

| Patent document cited in search report | Publication date (day/month/year) | Patent family member(s) | Publication date (day/month/year) |
|---|---|---|---|
| JP 2016-523959 A | 12 August 2016 | WO 2015/005859 A1<br>abstract, claims<br>JP 2016-525100 A<br>JP 2019-165747 A<br>US 2016/0159855 A1<br>US 2016/0159857 A1<br>US 2016/0207966 A1<br>US 2017/0320922 A1<br>US 2018/0244729 A1<br>WO 2015/005862 A1<br>EP 3019519 A1<br>EP 3019520 A1<br>CN 105377880 A<br>CN 105377881 A<br>CN 111848755 A | |
| JP 2013-256479 A | 26 December 2013 | US 2015/0152195 A1<br>abstract, claims, paragraph [0154]<br>WO 2013/187398 A1<br>EP 2862879 A1<br>CN 104603153 A | |
| WO 2012/165544 A1 | 06 December 2012 | US 2014/0179898 A1<br>abstract, paragraphs [0076]-[0077], [0087]-[0089], examples<br>EP 2728000 A1 | |
| JP 2010-156687 A | 15 July 2010 | US 2010/0221844 A1<br>claims, table I<br>JP 2012-229212 A<br>JP 2014-15464 A<br>JP 2017-31151 A<br>JP 2019-23188 A<br>JP 2021-4240 A<br>US 2018/0362595 A1<br>EP 2202310 A2<br>EP 2518151 A1<br>EP 2799550 A1<br>SG 162687 A<br>CN 101775069 A<br>ES 2406359 T<br>SG 195555 A<br>CN 103601794 A<br>ES 2464019 T<br>DK 2518151 T<br>CN 105111289 A<br>ES 2623889 T<br>CN 108864262 A<br>CN 108864263 A<br>CN 108912216 A | |
| CN 112210013 A | 12 January 2021 | US 2021/0032296 A1<br>claims, examples, fig. 5, 8 | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2007097361 A

- WO 2000023580 A

**Non-patent literature cited in the description**

- **TATIANA A. TATSUSOVA ; THOMAS L. MADDEN.** *FEMS Microbiol. Lett.,* 1999, vol. 174, 247-250

- **JANSON, J.-C.** Protein purification. 221-258